# EUROPEAN PATENT SPECIFICATION

(11) **EP 4 218 837 B1**
(45) Date of publication and mention of the grant of the patent: **27.11.2024**
(21) Application number: 23167679.2
(22) Date of filing: 17.11.2017
(51) Int. Cl.: A61L 9/013, A61L 9/04, A61L 9/14, A61L 9/12, C11D 3/50

(54) **VOLATILE COMPOSITIONS TO LIMIT OR ELIMINATE PERCEPTION OF FECAL MALODOUR**
FLÜCHTIGER ZUSAMMENSETZUNGEN ZUR BEGRENZUNG ODER BESEITIGUNG DER WAHRNEHMUNG VON FÄKALIENGERÜCHEN
COMPOSITIONS VOLATILES POUR LIMITER OU ÉLIMINER LA PERCEPTION DE MAUVAISES ODEURS FÉCALES

(30) Priority: 18.11.2016 US 201662424072 P; 13.04.2017 US 201762485060 P; 08.06.2017 EP 17175114; 11.09.2017 US 201762556714 P
(43) Date of publication of application: 02.08.2023
(62) Divisional of application: 17801694.5
(73) Proprietor: Firmenich SA, 1242 Satigny (CH)
(72) Inventor: MARGOT, Christian, 1211 Geneva (CH); ROGERS, Matthew, Plainsboro, 08536 (US); MARR, Gary, Plainsboro, 08536 (US); VUILLEUMIER, Christine, 1211 Geneva (CH); SMITH, Ben, Plainsboro, 08536 (US); CHAPUIS, Christian, 1211 Geneva (CH); STARKENMANN, Christian, 1211 Geneva (CH); CHAPPUIS, Charles, 1211 Geneva (CH); O'LEARY, Nicholas, Plainsboro, 08536 (US)
(74) Representative: Strych, Sebastian

(56) References cited:
- EP-A1- 2 962 678
- US-A1- 2004 223 871
- US-A1- 2015 005 213

## Description

### Technical Field

The present disclosure relates to the field of malodour counteraction. More particularly, it concerns compositions including a malodour antagonist system associated with perfuming ingredients performing as malodour counteractant, in a combination that significantly reduces the perception of fecal malodour. Such compositions, and their applications in consumer products are objects of the present disclosure.

### Background

Smells perceived as malodourous exist in many environments and are experienced in our daily life. The odourants eliciting this negative association can for example consist of commercial and residential environment malodours which can be generated by waste products, trash receptacles, toilets, cat litter, and food handling and processing. Toilet (in particular feces), kitchen and body malodour, are just a few of the common environmental sources of malodours in daily life. Malodours are usually complex mixtures of more than one malodourant compound which may typically include various amines, thiols, sulfides, short chain aliphatic and unsaturated acids, e.g. fatty acids, and their derivatives.

Residential or body related malodours are typically due to various chemical compounds such as indole, skatole, and methanethiol found in feces malodour; piperidine and morpholine found in urine; pyridine and triethyl amine found in kitchen and garbage malodours; geonol, 1-octen-3-ol, dimethyl disulfide, dimethyl trisulfide, 3-methyl-1-butanol found in laundry malodour; and short chain fatty acids, such as 3-methyl-3-hydroxyhexanoic acid, 3-methylhexanoic acid or 3-20 methyl-2-hexenoic acid, found in axillary malodours.

Such malodours are not pleasant for humans and therefore there is a constant need for malodour counteracting technologies (MOC) for decreasing or suppressing the perception of malodours. However the task is generally very difficult because the chemicals responsible for the malodour elicit extremely powerful smells and can have much lower detection thresholds than the odourants typically used to mask them. Therefore one has to use excessive amounts of MOC composition/compounds to achieve an acceptable malodour counteracting action.

Classes of compounds have been identified and reported as being useful for reducing the perception of certain malodours. For example US20100111889 describes a malodour control system suitable for use in disposable articles such as disposable cleaning wipes, baby wipes or skin care wipes, comprising an aldehyde, and ester, an ionone and a macrocyclic musk. Malodour neutralizing compositions containing acids and acyclic ketones have also been disclosed in US9774180. Other publications describe the use of compositions comprising ionones, irones and damascones in a similar context. Those classes of compounds have also been described as part of an odour masking base in personal care compositions -US2919440- or as part of a method of freshening air - US20040223871.

US 2015/0005213 A1 refers to the use as perfuming ingredient of 4-methyl-8-methylene-4,9-decadienal.

EP 2 962 678 A1 refers to flavour and fragrance compositions comprising selected acetophenone derivatives for dissolving flavours and fragrances.

There is still a need to find compositions that are efficient at lower concentrations in decreasing the perception of malodours. There is in particular a need for providing efficient products that would limit, decrease or eliminate the perception of toilet generated malodours, and in particular fecal malodour in order to promote public acceptance and use of toilets and discourage open defecation. The present disclosure provides a solution to the above mentioned problem by significantly enhancing the efficiency of class of ingredients known for their malodour counteraction by the addition of a malodour antagonist system, consisting of compounds that are blocking specific receptors of malodour targets.

### Summary

The present disclosure relates to a composition comprising a malodour antagonist system formed of ingredients that have been found to block specific receptors of fecal malodours including those disclosed in WO2014210585.

The combinations of the present disclosure have been found to provide unexpected results in terms of limitation or elimination of the perception of fecal malodour.

A malodour counteracting composition as described in claim 1 is an object of the present disclosure.

A perfumed consumer product as described in claim 6, and comprising an effective amount of a malodour counteracting composition as defined above is another object of the present disclosure.

### Brief Description of the Drawings

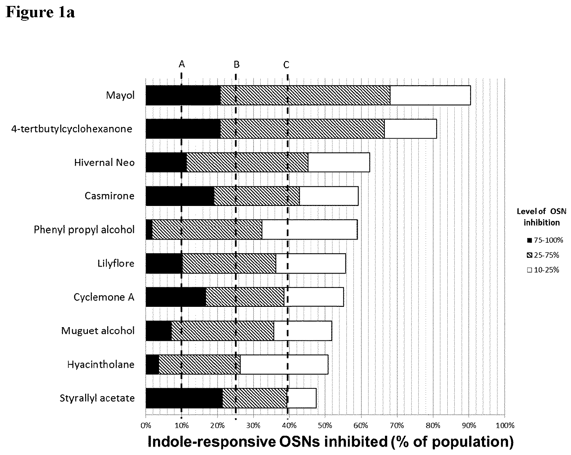

Figure 1a shows results of live neuron assay antagonism screening against indole malodour target.

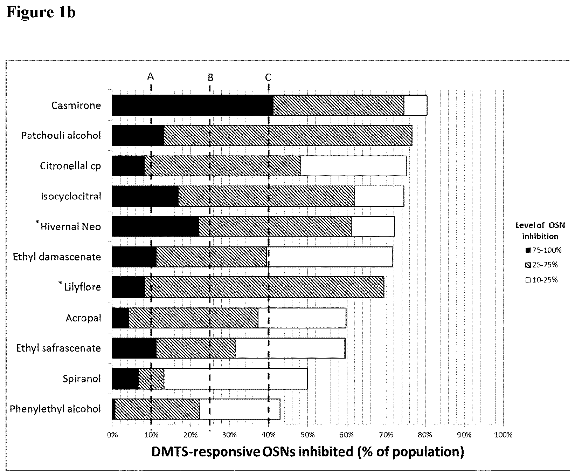

Figure 1b shows results of live neuron assay antagonism screening against dimethyl trisulfide (also referred to as DMTS) malodour target.

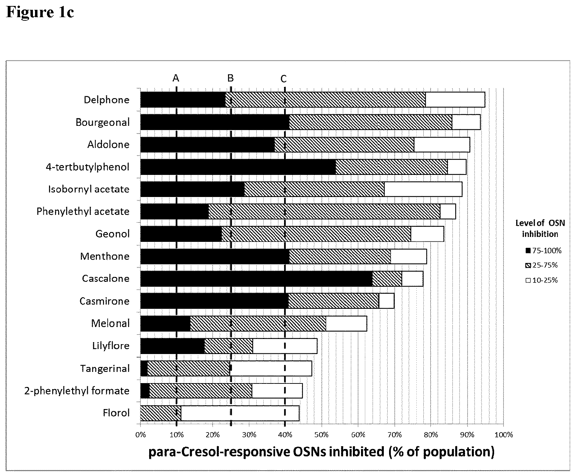

Figure 1c shows results of live neuron assay antagonism screening against p-cresol malodour target.

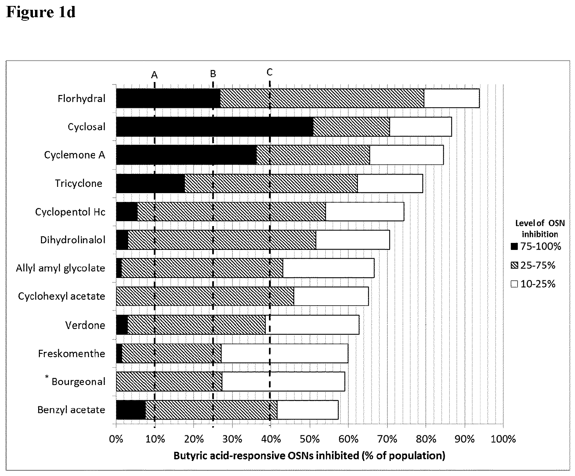

Figure 1d shows results of live neuron assay antagonism screening against butyric acid malodour target.

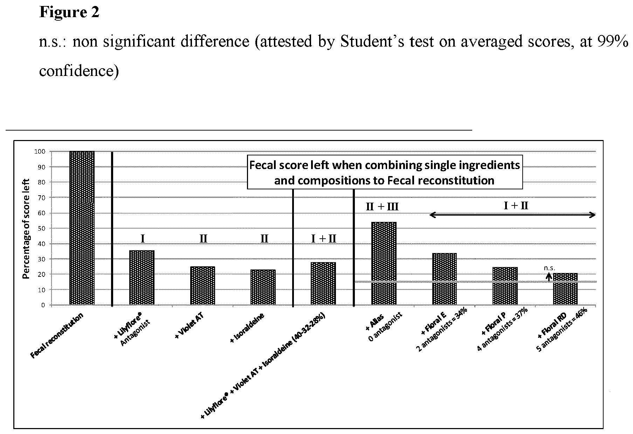

Figure 2 reports the results of fecal score remaining when combining a malodour antagonist system consisting of (2,5-dimethyl-2,3-dihydro-1H-inden-2-yl)methanol (also referred as LILYFLORE^{®}), with a functional perfume accord consisting of α-ionone (also referred to as Violet AT) and isoraldeine (consisting of isomethyl-alpha-ionone and alpha-methylionone).

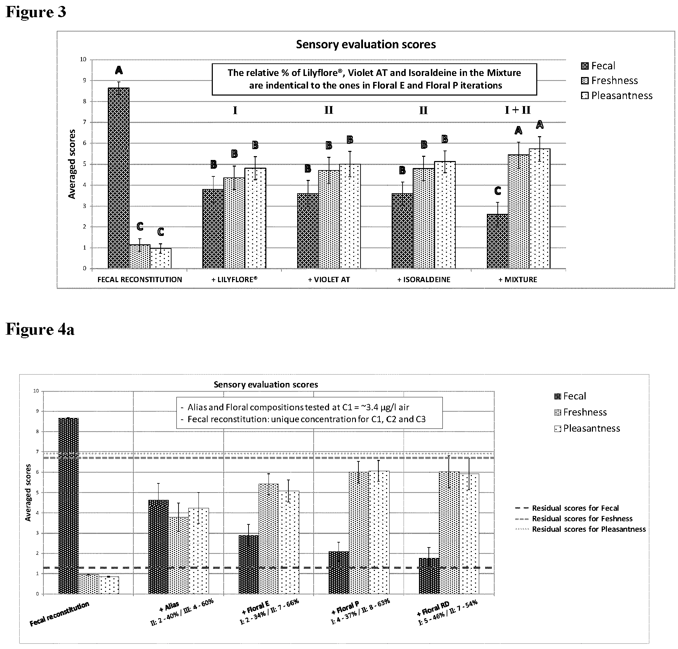

Figure 3 shows the performance of the 3 single compounds performance and of their mixture (a floral accord) against the fecal malodour reconstitution.

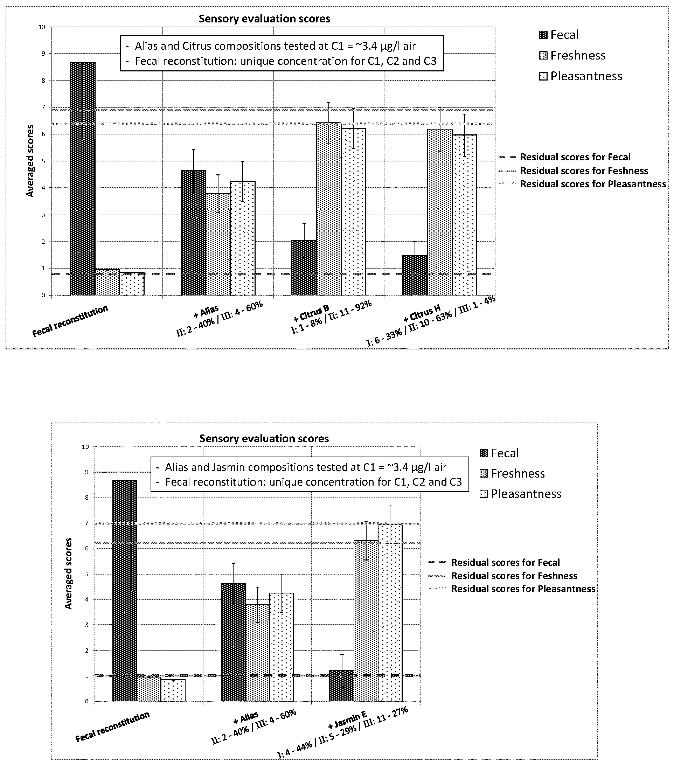

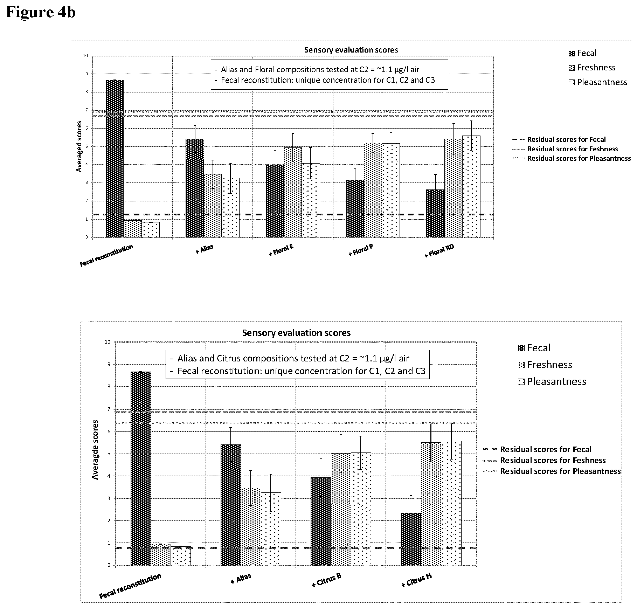

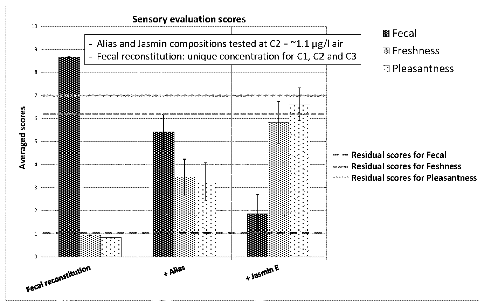

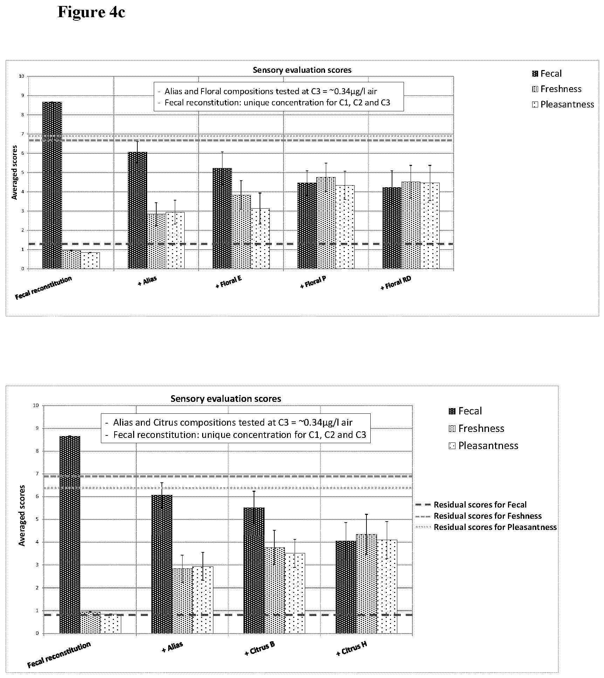

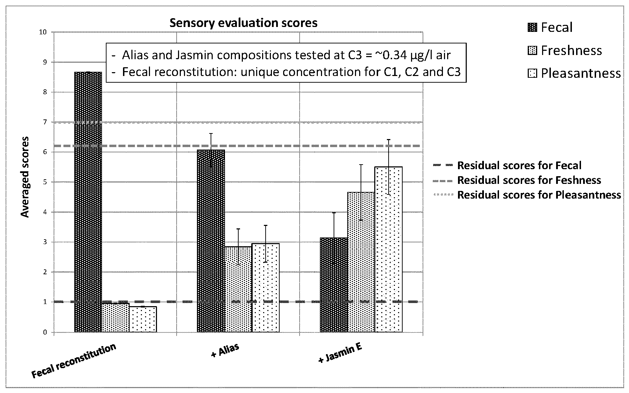

Figure 4 shows graphs that represent the scores of Fecal, Freshness and Pleasantness attributes for the fecal reconstitution alone (at a single concentration across all tests) and for the combination of different compositions.
   - Figure 4a (i) (ii) (iii): Composition tested at 3.4 µg/l (C1) air against the fecal reconstitution (i) floral; (ii) citrus; (iii) jasmin;
   - Figure 4b: Composition tested at 1.1 µg/l (C2) air against the fecal reconstitution;
   - Figure 4c: Composition tested at 0.33 µg/l (C3) air against the fecal reconstitution.

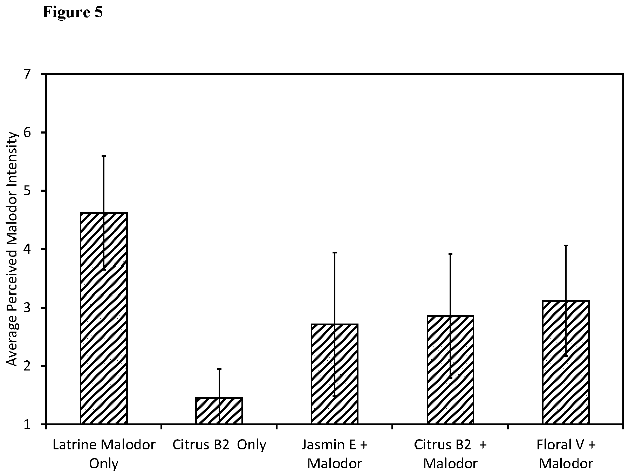

Figures 5-10 represent mean malodour intensity measured in cabins.

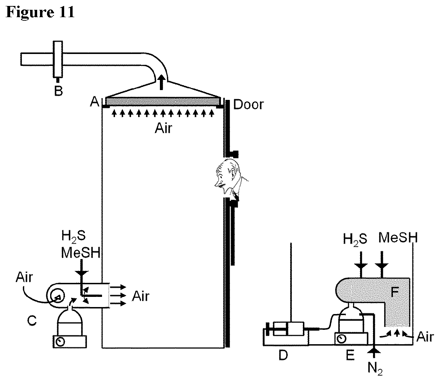

Figure 11 depicts a model latrine. Left, Side view diagram of a model latrine. A, Laminar filter. B, Damper. C, Odour generator. Right, Front view of the odour generator placed behind the model latrine. D, Syringe pump. E, Round-bottom glass mounted on the heating plate. (F) Air inlet pipe that guides the air carrying the odour treatments inside the model latrine.

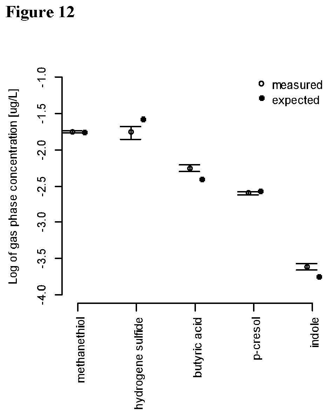

Figure 12 shows the relationship between the predicted amounts and the measured amounts of the constituents on the toilet malodour in the model latrine. Odourant homogeneity within a toilet and between three toilets. Mean ± standard deviation (SD) of measured gas phase concentrations compared with the expected values. N = 9.

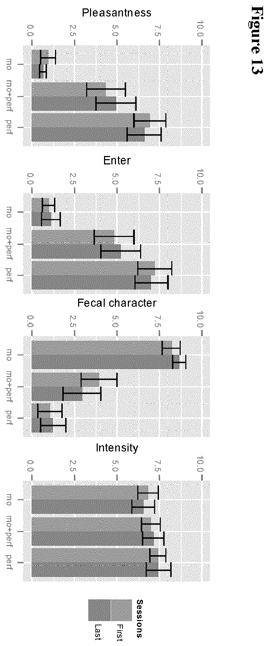

Figure 13 shows sensory data, showing the effect of a perfume composition according to the present disclosure on perceived pleasantness, desire to enter, fecal character and fecal intensity in a model latrine. Validation of the sensory protocol. Mean ± 95% confidence interval (CI) of pleasantness, willingness to enter, fecal character, and intensity of odour ratings. Mo: malodour. Perf: 4.9 µg/l perfume formulation Floral D - see Table 14.

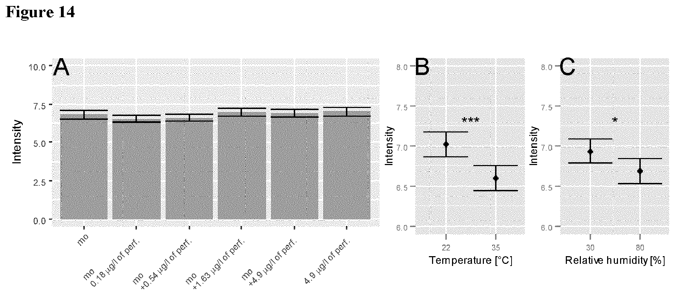

Figure 14 A) Evaluation of the intensity of the sensory stimuli with a constant Mukuru fecal reconstitution malodour and increasing perfume concentration. Mean ± 95% CI of the intensity as a function of odour treatments, temperature, and relative humidity. B) Intensity evaluation at 22 °C and 35 °C; the data at 30% and 80% humidity are combined. C) Intensity evaluation at 30% and 80% humidity; the data at 22 °C and 35 °C are combined. Asterisks show the levels of significant differences in means, ***P < 0.0001, *P < 0.05.

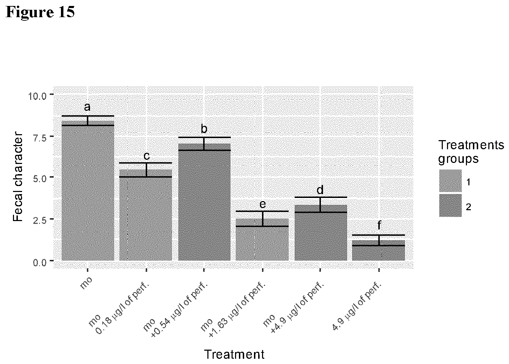

Figure 15. Sensory evaluation of the impact of the reference malodour or perfume on the response variable, fecal character. Mean ± 95% CI of the fecal character of the odour treatments. Blue indicates the groups of odour compared with malodour and pink indicates the groups compared with perfume. Means with different letters are significantly different following a pairwise test based on ANOVA.

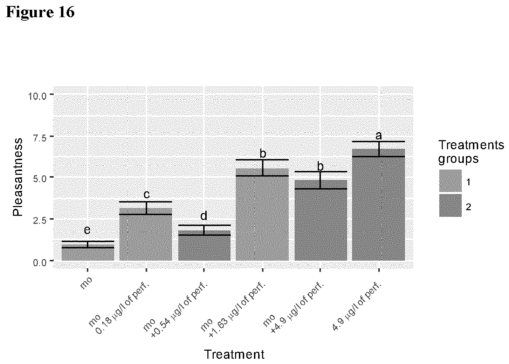

Figure 16. Sensory evaluation of the impact of the reference malodour or perfume on the response variable, pleasantness. Mean ± 95% CI of the pleasantness ratings as a function of the odour treatments. Blue indicates the groups of odour compared with malodour and pink indicates the groups compared with perfume. Means with different letters are significantly different following a pairwise test based on ANOVA.

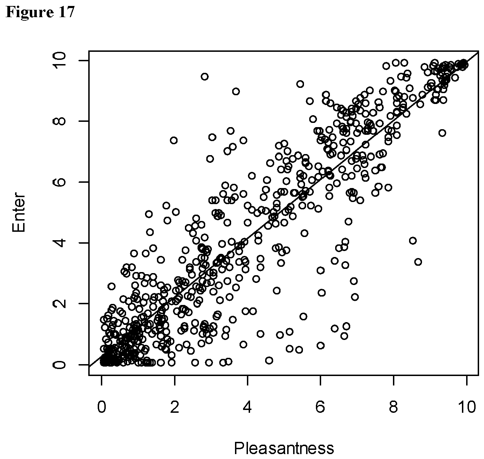

Figure 17. The enter ratings as a function of the pleasantness ratings. The line shows the linear model that predicts the ratings by the pleasantness ratings.

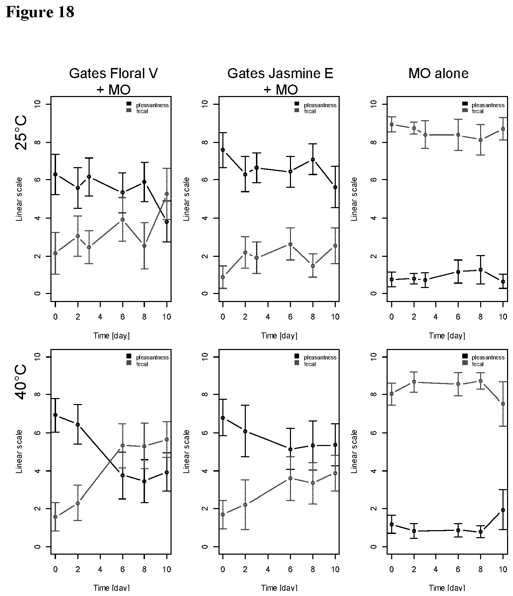

Figure 18 shows the mean ± 95% confidence interval (CI) of the pleasantness (black line) and fecal character (gray line) ratings in test latrines as a function of time for the Mukuru fecal reconstitution malodour (MO) + Floral V, the malodour + Jasmine E and the Mukuru fecal reconstitution malodour alone at 25°C (top three graphs) and 40°C (bottom three graphs).

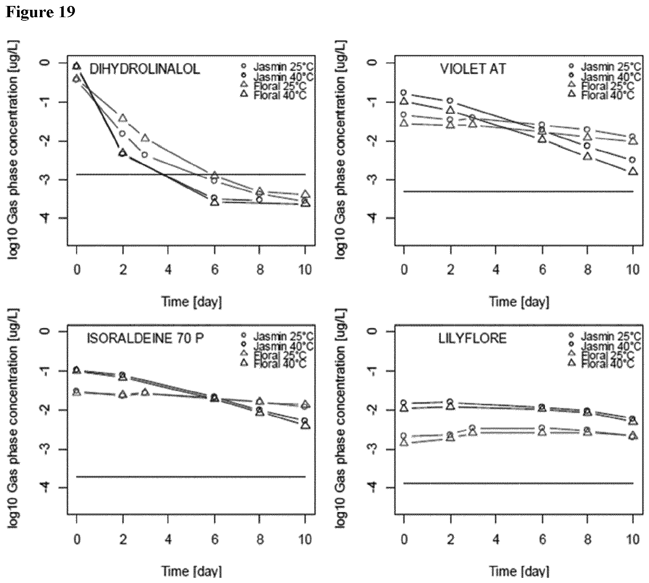

Figure 19 shows the mean of the gas phase concentrations as a function of time for the antagonist compounds indicated in the Floral V formulation (triangles), and Jasmine E formulation (circle), observed at 25°C (Dark lines) and 40°C(light lines). Horizontal solid lines are the ODT.

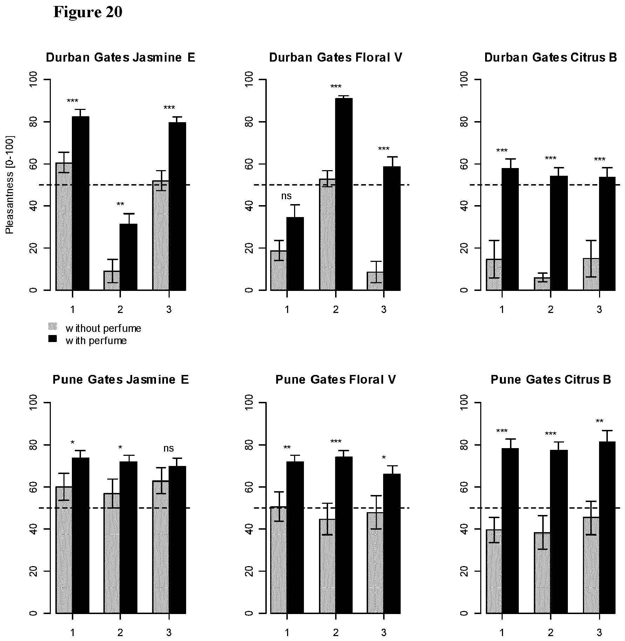

Figure 20 shows the mean ± SEM of the pleasantness ratings for the test formulations Jasmine E (left vertical row); Floral V (middle vertical row); and Citrus 259389 B (right vertical row) in both countries (Top Row: Durban, South Africa; bottom Row: Pune, India). The numbers 1, 2, 3 correspond to the three latrines tested. Stars showed significant differences in ratings obtained without and with treatments: ns P>0.05; * P<=0.05; ** P<0.01; *** P<0.001. The black bars denote the pleasantness ratings observed for the test formulation. The grey bars denote the pleasantness ratings observed in the absence of the test formulation.

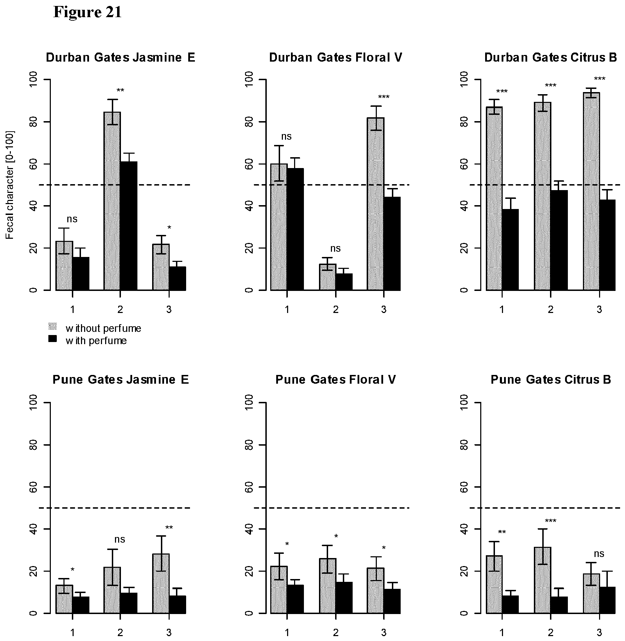

Figure 21 shows the mean ± SEM of the fecal character ratings for the test formulations Jasmine E (left vertical row); Floral V (middle vertical row); and Citrus 259389 B (right vertical row) in both countries (Top Row: Durban, South Africa; bottom Row: Pune, India). The numbers 1, 2, 3 correspond to the three latrines tested. Stars showed significant differences in ratings obtained without and with treatments: ns P>0.05; * P<=0.05; ** P<0.01; *** P<0.001. The black bars denote the fecal character ratings observed for the test formulation. The grey bars denote the fecal character ratings observed in the absence of the test formulation.

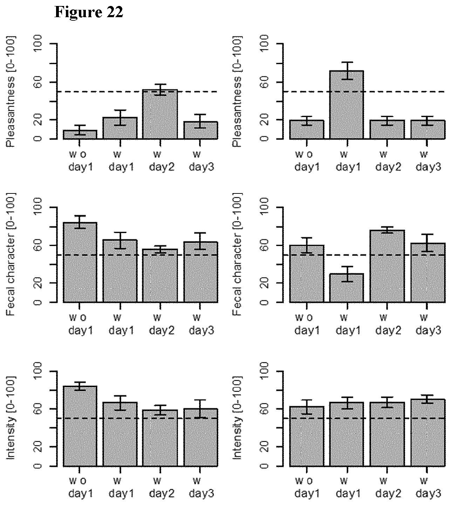

Figure 22 shows the mean ± SEM of the pleasantness, fecal character and intensity ratings as a function of time for two individual latrines in Durban. "WO" without test formulation (baseline). "W" with test formulation. The left vertical column denotes the observed values in latrine no. 1, treated with or without the Jasmine E formulation. The right vertical column denotes the observed values in latrine no. 2, treated with or without the Floral V formulation.

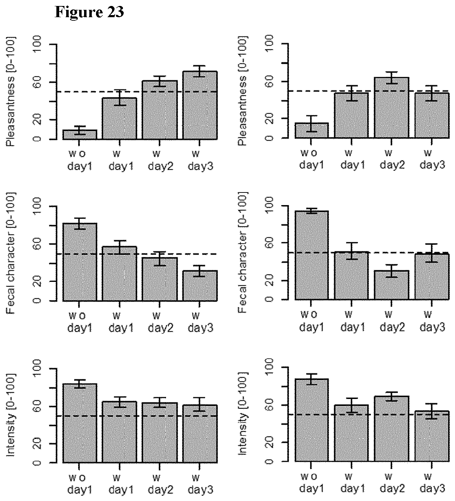

Figure 23 shows the mean ± SEM of the pleasantness, fecal character and intensity ratings as a function of time for two individual latrines in Durban. "WO" without test formulation (baseline). "W" with test formulation. The left vertical column denotes the observed values in latrine no. 3, treated with or without the Floral V formulation. The right vertical column denotes the observed values in latrine no. 2, treated with or without the Citrus 259389 B formulation.

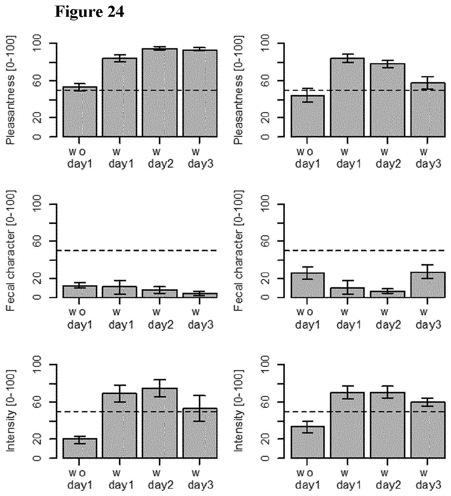

Figure 24 shows the mean ± SEM of the pleasantness, fecal character and intensity ratings as a function of time for two individual latrines in Durban and Pune. "WO" without test formulation (baseline). "W" with test formulation. The left vertical column denotes the observed values in latrine no. 2, treated with or without the Floral V formulation in Durban. The right vertical column denotes the observed values in latrine no. 2, treated with or without the Floral V formulation in Pune.

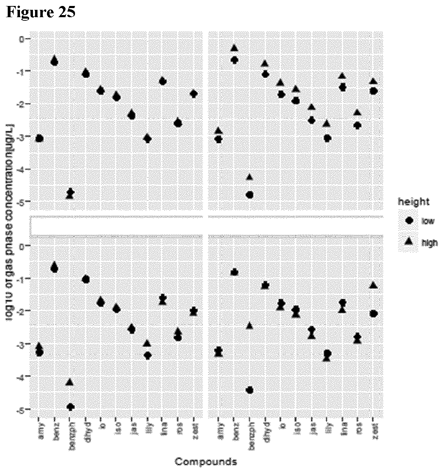

Figure 25 shows the observed gas phase concentrations of compounds found in the air samples collected at two different heights in each toilet. "low", 0.15-0.3m; "high", 1.5-1.7m. "amy" amylcinnamic aldehyde; "benz" benzyl acetate; "benzph" benzylphenyl acetate; "dihyd" dihydrolinalol; "io" α-ionone; "iso" isoraldeine; "jas" cis jasmone; "lily" lyliflore; "lina" linalyl acetate;"ros" rosinol; "zest" zestover. The upper left panel denotes the values observed in latrine no. 2 in Durban. The upper right panel denotes the values observed in latrine no. 3 in Durban. The lower left panel denotes the values observed in latrine no. 1 in Pune. The lower right panel denotes the values observed in latrine no. 2 in Pune.

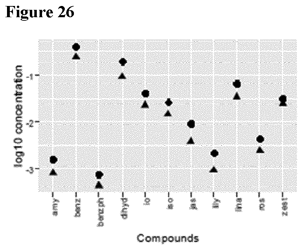

Figure 26 gas phase concentrations (log10 of µg/L) of compounds found in the air samples collected on the field (triangle) and in model latrines (circle), "amy" amylcinnamic aldehyde; "benz" benzyl acetate; "benzph" benzylphenyl acetate; "dihyd" dihydrolinalol; "io" α-ionone; "iso" isoraldeine; "jas" cis jasmone; "lily" lyliflore; "lina" linalyl acetate;"ros" rosinol; "zest" zestover.

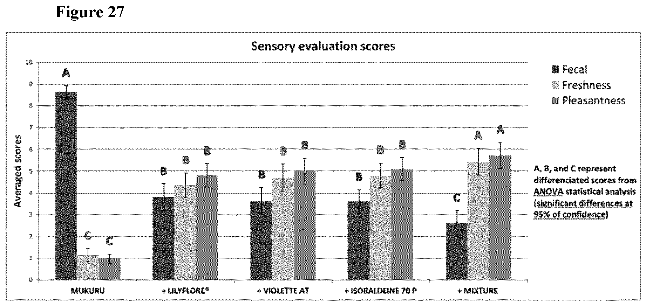

Figure 27 shows the average attribute scores for a test and three control formulations evaluated in combination with a Mukuru fecal reconstitution malodour.

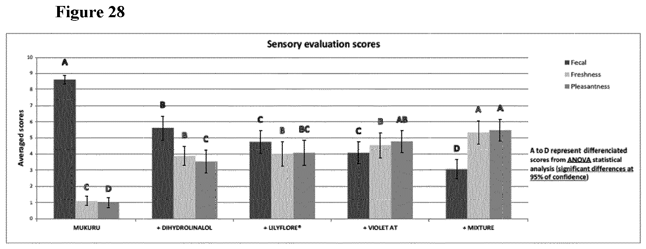

Figure 28 shows the average attribute scores for a test and three control formulations evaluated in combination with a Mukuru fecal reconstitution malodour.

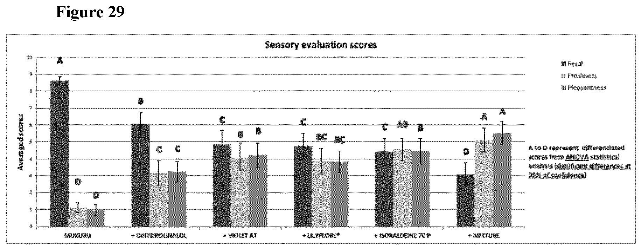

Figure 29 shows the average attribute scores for a test and four control formulations evaluated in combination with a Mukuru fecal reconstitution malodour.

### Detailed Description

### Definitions

Unless otherwise indicated, percentages are meant to designate percentages by weight.

As used herein, the terms include or comprise are meant to be non-limiting.

As used herein, the terms malodour receptor antagonist, malodour antagonist system or malodour antagonist ingredient, also referred to as group I is meant to designate one or several compounds that each have the capacity to inhibit at least one olfactory receptor that responds to a malodour target, identified by measuring activity of olfactory neurons or isolated receptors in cultured cell lines whose responses are driven by receptors as described under the examples below.

As used herein, "malodour target" is meant to designate a molecular component of fecal malodour characterized in Lin et al, Environ. Sci. Technol., 2013, 47 (14), pp 7876-7882, including indole, butyric acid, p-cresol, skatole, and dimethyl trisulfide.

As used herein, the term functional perfume accord (referred to as group II) is meant to designate a mixture of at least two perfuming ingredients, referred as functional perfuming ingredients which have been established through e.g. sensory measurement as performing against at least one element of a fecal malodour.

As used herein, the term non-functional perfume accord (referred to as group III) is meant to be a mixture of at least one, alternatively, at least two perfuming ingredients, referred to as non-functional perfuming ingredients that are not performing as fecal malodour counteractant, i.e. perfuming ingredients that are not part of group I or group II .

As used herein, the term perfume or perfume oil or perfume accord are used to designate a mixture of perfuming ingredients.

Moreover, by "perfuming ingredient" it is meant here a compound, which can be used in a perfuming preparation or a composition to impart at least an hedonic effect. In other words such an ingredient, to be considered as being a perfuming one, must be recognized by a person skilled in the art of perfumery as being able to impart or modify in a positive or pleasant way the odour of a composition, and not just as having an odour.

The nature and type of the perfuming ingredients do not warrant a more detailed description here, which in any case would not be exhaustive, the skilled person being able to select them on the basis of their general knowledge and according to intended use or application and the desired organoleptic effect. In general terms, these perfuming ingredients belong to chemical classes as varied as alcohols, lactones, aldehydes, ketones, esters, ethers, acetates, nitriles, terpenoids, nitrogenous or sulphurous heterocyclic compounds and essential oils, and the perfuming co-ingredients can be of natural or synthetic origin.

In particular one may cite perfuming ingredients which are commonly used in perfume formulations, such as:
- Aldehydic ingredients: decanal, dodecanal, 2-methyl-undecanal, 10-undecenal, octanal and/or nonenal;
- Aromatic-herbal ingredients: eucalyptus oil, camphor, eucalyptol, menthol and/or alpha-pinene;
- Balsamic ingredients: coumarine, ethylvanillin and/or vanillin;
- Citrus ingredients: dihydromyrcenol, citral, orange oil, linalyl acetate, citronellyl nitrile, orange terpenes, limonene, 1-P-menthen-8-yl acetate and/or 1,4(8)-P-menthadiene;
- Floral ingredients: Methyl dihydrojasmonate, linalool, Citronellol, phenylethanol, 3-(4-tert-butylphenyl)-2-methylpropanal, hexylcinnamic aldehyde, benzyl acetate, benzyl salicylate, tetrahydro-2-isobutyl-4-methyl-4(2H)-pyranol, beta ionone, methyl 2-(methylamino)benzoate, (E)-3-methyl-4-(2,6,6-trimethyl-2-cyclohexen-1-yl)-3-buten-2-one, hexyl salicylate, 3,7-dimethyl-1,6-nonadien-3-ol, 3-(4-isopropylphenyl)-2-methylpropanal, verdyl acetate, geraniol, P-menth-1-en-8-ol, 4-(1,1-dimethylethyl)-1-cyclohexyle acetate, 1,1-dimethyl-2-phenylethyl acetate, 4-cyclohexyl-2-methyl-2-butanol, amyl salicylate, high cis methyl dihydrojasmonate, 3-methyl-5-phenyl-1-pentanol, verdyl proprionate, geranyl acetate, tetrahydro linalool, cis-7-P-menthanol, Propyl (S)-2-(1,1-dimethylpropoxy)propanoate, 2-methoxynaphthalene, 2,2,2-trichloro-1-phenylethyl acetate, 4/3-(4-hydroxy-4-methylpentyl)-3-cyclohexene-1-carbaldehyde, amylcinnamic aldehyde, 4-phenyl-2-butanone, isononyle acetate, 4-(1,1-dimethylethyl)-1-cyclohexyl acetate, verdyl isobutyrate and/or mixture of methylionones isomers;
- Fruity ingredients: gamma undecalactone, 4-decanolide, ethyl 2-methyl-pentanoate, hexyl acetate, ethyl 2-methylbutanoate, gamma nonalactone, allyl heptanoate, 2-phenoxyethyl isobutyrate, ethyl 2-methyl-1,3-dioxolane-2-acetate and/or diethyl 1,4-cyclohexane dicarboxylate;
- Green ingredients: 2,4-Dimethyl-3-cyclohexene-1-carbaldehyde, 2-tert-butyl-1-cyclohexyl acetate, styrallyl acetate, allyl (2-methylbutoxy)acetate, 4-methyl-3-decen-5-ol, diphenyl ether, (Z)-3-hexen-1-ol and/or 1-(5,5-dimethyl-1-cyclohexen-1-yl)-4-penten-1-one;
- Musk ingredients: 1,4-dioxa-5,17-cycloheptadecanedione, pentadecenolide, 3-Methyl-5-cyclopentadecen-1-one, 1,3,4,6,7,8-hexahydro-4,6,6,7,8,8-hexamethyl-cyclopenta-g-2-benzopyrane, (15,1'R)-2-[1-(3',3'-dimethyl-1'-cyclohexyl)ethoxy]-2-methylpropyl propanoate, pentadecanolide and/or (15,1'R)-[1-(3',3'-Dimethyl-1'-cyclohexyl)ethoxycarbonyl]methyl propanoate;
- Woody ingredients: 1-(octahydro-2,3,8,8-tetramethyl-2-naphtalenyl)-1-ethanone, patchouli oil, terpenes fractions of patchouli oil, (1'R,E)-2-ethyl-4-(2',2',3'-trimethyl-3'-cyclopenten-1'-yl)-2-buten-1-ol, 2-ethyl-4-(2,2,3-trimethyl-3-cyclopenten-1-yl)-2-buten-1-ol, Methyl cedryl ketone, 5-(2,2,3-trimethyl-3-cyclopentenyl)-3-methylpentan-2-ol, 1-(2,3,8,8-tetramethyl-1,2,3,4,6,7,8,8a-octahydronaphthalen-2-yl)ethan-1-one and/or isobornyl acetate;
- Other ingredients (e.g. amber, powdery spicy or watery): dodecahydro-3a,6,6,9a-tetramethyl-naphtho[2,1-b]furan and any of its stereoisomers, heliotropin, anisic aldehyde, eugenol, cinnamic aldehyde, clove oil, 3-(1,3-benzodioxol-5-yl)-2-methylpropanal and/or 3-(3-isopropyl-1-phenyl)butanal.

Perfuming ingredients may not be limited to the above mentioned, and many other of these ingredients are in any case listed in reference texts such as the book by S. Arctander, Perfume and Flavor Chemicals, 1969, Montclair, New Jersey, USA, or its more recent versions, or in other works of a similar nature, as well as in the patent literature in the field of perfumery. It is also understood that co-ingredients may also be compounds known to release in a controlled manner various types of perfuming compounds.

It has now been surprisingly established that the association of a malodour receptor antagonist system comprising at least one ingredient selected from the group of Table 1 with a functional perfume accord consisting of perfuming ingredients performing against fecal malodour, improves the effect of the functional perfume accord in limiting, decreasing or eliminating the perception of fecal malodour.

A first object according to the present disclosure is therefore a malodour counteracting composition comprising:
(i) from about 2 wt% to about 85 wt%, of a malodour receptor antagonist system comprising at least one ingredient selected from the group of Table 1;
(ii) from about 15 wt% to 98 wt% of a functional perfume accord comprising at least 2 perfuming ingredient(s) selected from Table 2, characterized in that the malodour receptor antagonist system comprises (2,5-dimethyl-2,3-dihydro-1H-inden-2-yl)methanol, and the functional perfume accord comprises citral, isoraldeine and α-ionone.

**Table 1: Malodour receptor antagonists - Group I**

| Common Name | Chemical name |
|---|---|
| ACETAROLLE | (1RS,6RS,11RS)-2,2,9,11-tetramethylspiro[5.5]undec-8-en-1-yl acetate |
| BENZYL ACETATE | Benzyl Acetate |
| PHENYLETHYL ACETATE | 2-PHENYLETHYL ACETATE |
| ISOBORNYL ACETATE | (1R,2R)-1,7,7-TRIMETHYL-BICYCLO[2.2.1]HEPT-2-YL ACETATE |
| ACROPAL | 3-(4-METHYL-3-PENTENYL)-3-CYCLOHEXEN E-1-CARBALDEHYDE (A) + 4-(4-METHYL-3-PENTENYL)-3-CYCLOHEXENE-1-CARBALDEHYDE |
| ALDOLONE | 7-PROPYL-2H,4H-1,5-BENZODIOXEPIN-3-ONE |
| ALLYL AMYL GLYCOLATE | ALLYL (3-METHYLBUTOXY)ACETATE (A) + (+-)-ALLYL (2-METHYLBUTOXY)ACETATE (B) |
| AMBERWOOD | (ETHOXYMETHOXY)CYCLODODECANE |
| AMIONE | (+-)-(1E)-1-(2,6,6-trimethyl-2-cyclohexen-1-yl)-1,6-heptadien-3-one (A) + (1E)-1-(2,6,6-trimethyl-1-cyclohexen-1-yl)-1,6-heptadien-3-one (B) |
| TETRAMETHYL ETHYL CYCLOHEXENONE | 3,5-DIETHYL-5,6-DIMETHYL-2-CYCLOHEXEN-1-ONE (A) + 3,5-DIETHYL-2,5-DIMETHYL-2-CYCLOHEXEN-1-ONE (B) |
| BOURGEONAL | 3-(4-TERT-BUTYLPHENYL)PROPANAL |
| CACHALOX | 3aRS,5aSR,9aSR,9bSR)-3a,6,6,9a-tetramethyldodecahydronaphtho[2,1-b]furan |
| CASCALONE^{®} | 7-ISOPROPYL-2H,4H-1,5-BENZODIOXEPIN-3-ONE |
| CASMIRONE | (4E,8E)-4,8-cyclododecadien-1-one (A) + (4E,8Z)-4,8-cyclododecadien-1-one (B) + (4Z,8E)-4,8-cyclododecadien-1-one (C) |
| CITRONELLAL CP | (+)-(R)-3,7-DIMETHYL-6-OCTENAL |
| VETIKOLACETATE | (+-)-1,3-DIMETHYL-3-PHENYLBUTYL ACETATE |
| CYCLEMONE A | 1,2,3,4,5,6,7,8-OCTAHYDRO-8,8-DIMETHYL-2-NAPHTHALENECARBALDEHYDE (A) + (B,C,D) + OCTAHYDRO-5,5-DIMETHYL-2-NAPHTHALENECARBALDEHYDE |
| CYCLOPENTOL HC | (+-)-CIS-2-PENTYL-1-CYCLOPENTANOL |
| CYCLOSAL | (+-)-3-(4-isopropylphenyl)-2-methylpropanal |
| ETHYL DAMASCENATE | ETHYL 2,6,6-TRIMETHYL-1,3-CYCLOHEXADIENE-1-CARBOXYLATE |
| DELPHONE | (+-)-2-pentyl cyclopentanone |
| DIHYDROLINALOL | (+-)-3,7-DIMETHYL-1-OCTEN-3-OL |
| DYNASCONE | 1-(5,5-dimethyl-1-cyclohexen-1-yl)-4-penten-1-one |
| BENZYL FORMATE | BENZYL FORMATE |
| PHENYLETHYL FORMATE | 2-PHENYLETHYL FORMATE |
| FRUCTALATE | DIETHYL 1,4-CYCLOHEXANEDICARBOXYLATE |
| FRUCTOPYRIDINE | 3-(2,2-DIMETHYLPROPYL)PYRIDINE |
| GLYCOLIERRAL | (1RS,2SR,8RS)-2-(8-ISOPROPYL-6-METHYL-BICYCLO[2.2.2]OCT-5-EN-2-YL)-1,3-DIOXOLANE |
| HIVERNAL^{®} NEO | 3-(3,3-dimethyl-2,3-dihydro-1H-inden-5-yl)propanal (A) + 3-(1,1-dimethyl-2,3-dihydro-1H-inden-4-yl)propanal (B) + 3-(1,1-dimethyl-2,3-dihydro-1H-inden-5-yl)propanal (C) |
| ISOCYCLOCITRAI | 3,5,6-TRIMETHYL-3-CYCLOHEXEN E-1-CARBALDEHYDE (A) + 2,4,6-TRIMETHYL-3-CYCLOHEXENE-1-CARBALDEHYDE (B) |
| ISOBUTYLQUINOLEINE | 2-ISOBUTYLQUINOLINE |
| ISOPROPYLQUINOLEINE | 6(8)-ISOPROPYLQUINOLINE |
| LILYFLORE^{®} | (+-)-2,5-DIMETHYL-2-INDANMETHANOL |
| MELONAL | (+-)-2,6-DIMETHYL-5-HEPTENAL |
| MENTHONE | (2RS,5SR)-5-methyl-2-(2-propanyl)cyclohexanone (A) + (2RS,5RS)-5-methyl-2-(2-propanyl)cyclohexanone (B) |
| MUSCONE LAEVO | (-)-(3R)-3-METHYL-1-CYCLOPENTADECANON E |
| OXYCARYOPHYLLEN E | 4,12,12-TRIM ETHYL-9-M ETHYLEN E-5-OXATRICYCLO[8.2.0.0(4,6)]DODECANE |
| ORIVOL | 4-(1,1-DIMETHYLPROPYL)CYCLOHEXANOL |
| PHENETHYLOL ORD | 2-PHENYLETHANOL |
| PLICATONE | (1RS,2SR,5RS,7RS,8SR)-5-methyltricyclo[6.2.1.0~2,7~]undecan-4-one (A) + (1RS,2SR,5SR,7RS,8SR)-5-methyltricyclo[6.2.1.0~2,7~]undecan-4-one (B) |
| ROSINOL CRYST | (+-)-2,2,2-TRICHLORO-1-PHENYLETHYL ACETATE |
| ETHYL SAFRASCENATE | ETHYL 4,6,6-TRIMETHYL-1,3-CYCLOHEXADIENE-1-CARBOXYLATE |
| SALVIAC | (+-)-(6RS,10RS)-2,2,8,10-tetramethylspiro[5.5]undec-8-en-1-one (A) + (+-)-(6RS,10SR)-2,2,8,10-tetramethylspiro[5.5]undec-8-en-1-one (B) + (6RS,7RS)-2,2,7,9-tetramethylspiro[5.5]undec-8-en-1-one (C) + (6RS,7SR)-2,2,7,9-tetramethylspiro[5.5]undec-8-en-1-one (D) |
| SPIRANOL | (5RS,6RS)-2,6,10,10-TETRAMETHYL-1-OXASPIRO[4.5]DECAN-6-OL |
| TANGERINAL | (4Z)-4-dodecenal |
| TERRANOL | 2,2,7,7-tetramethyltricyclo[6.2.1.0~1,6~]undecan-6-ol |
| TRIMOFIX | 1-(2,6,10-TRIMETHYL)-1-(2,5,9-CYCLODODECATRIEN-1-YL)-1-ETHANONE + 1-(2,6,10-TRIMETHYL)-1-(1,5,9-CYCLODODECATRIEN-1-YL)-1-ETHANONE + 1-(6,10-DIMETHYL, 2-METHYLENE)-1-(2,5,9-CYCLODODECATRIEN-1-YL)-1-ETHANONE |
| WOLFWOOD | (+)-(1S,2S,3S,5R)-2,6,6-trimethylspiro[bicyclo[3.1.1]heptane-3,1'-cyclohexane]-2'-en-4'-one |
| 3-PHENYL-1-PROPANOL | 3-PHENYL-1-PROPANOL |
| MUGUET ALCOHOL | 2,2-DIMETHYL-3-PHENYL-1-PROPANOL |
| CEDRENOL | (+-)-3,6,8,8-tetramethyloctahydro-1H-3a,7-methanoazulen-6-ol |
| CEDROXYDE | (+-)-(4Z,8E)-1,5,8-trimethyl-13-oxabicyclo[10.1.0]trideca-4,8-diene (A) + (+-)-(4Z,8E)-1,4,8-trimethyl-13-oxabicyclo[10.1.0]trideca-4,8-diene (B) |
| GEONOL | (+-)-PERHYDRO-4alpha,8Abeta-DIMETHYL-4A-NAPHTHALENOL |
| HYACINTHOLANE | (+-)-2,2-dimethyl-4-phenyl-1,3-dioxolane |
| MAGNOLAN | (+-)-2,4-dimethyl-4,4a,5,9b-tetrahydroindeno[1,2-d][1,3]dioxine (ISOMER A) + (+-)-2,4-dimethyl-4,4a,5,9b-tetrahydroindeno[1,2-d][1,3]dioxine (ISOMER B) (A + B) |
| PATCHOULI ALCOHOL | (-)-(3R,6S,8S)-2,2,6,8-tetramethyltricyclo[5.3.1.0~3,8~]undecan-3-ol |
| PATCHOULI OIL | PATCHOULI OIL |
| PHENYLETHYL SALICYLATE | 2-PHENYLETHYL 2-HYDROXYBENZOATE |
| 4-TERT BUTYLPHENOL | 4-TERT BUTYLPHENOL |
| CYCLOHEXYL ACETATE | (1RS,2RS)-2-(2-methyl-2-propanyl)cyclohexyl acetate (A) + (1RS,2SR)-2-(2-methyl-2-propanyl)cyclohexyl acetate (B) |
| STYRALLYL ACETATE | (+-)-1-PH ENYLETHYL ACETATE |
| 3,5,5-TRIETHYL-2,4,6-TRIMETHYL-2-CYCLOHEXEN-1-ONE | (4RS,6SR)-3,5,5-triethyl-2,4,6-trimethyl-2-cyclohexen-1-one (A) + (4RS,6RS)-3,5,5-triethyl-2,4,6-trimethyl-2-cyclohexen-1-one (B) |
| CEDRENE EPOXYDE | 8,9-epoxycedrane |
| CYCLODODECANONE | CYCLODODECANONE |
| EXALTENONE | (Z)-4-CYCLOPENTADECEN-1-ONE |
| FLORHYDRAL | (+-)-3-(3-ISOPROPYL-1-PHENYL)BUTANAL |
| FLOROL | (+-)-TETRAHYDRO-2-ISOBUTYL-4-METHYL-4(2H)-PYRANOL |
| FRESKOMENTHE | 2-(1-METHYLPROPYL)-1-CYCLOH EXANON E |
| MARITIMA | 4-(4,8-DIMETHYL-3,7-NONADIEN-1-YL)PYRIDINE |
| MAYOL | [cis-4-(2-propanyl)cyclohexyl]methanol |
| MUSCENONE DEXTRO | (+)-(3R,5Z)-3-methyl-5-cyclopentadecen-1-one |
| MUSCONE | (+-)-3-methylcyclopentadecanone |
| NOOTKETONE | (+)-(4R,4aS,6R)-4,4a-dimethyl-6-(1-propen-2-yl)-4,4a,5,6,7,8-hexahydro-2(3H)-naphthalenone |
| PALISANDIN | METHOXYCYCLODODECANE |
| PARA TERT BUTYLCYCLOHEXANONE | 4-(2-methyl-2-propanyl)cyclohexanone |
| PINOACETALDEHYDE | 3-(6,6-DIMETHYL-BICYCLO[3.1.1]HEPT-2-EN-2-YL)PROPANAL |
| PRODUCT AC | cedran-8-yl acetate |
| RHUBOFURAN | (+-)-2,4-dimethyl-4-phenyltetrahydrofuran |
| SAFRALEINE | (+-)-2,3,3-TRIMETHYL-1-INDANONE |
| TERT-BUTYLPHENOL, 2- | 2-TERT-BUTYLPHENOL |
| TRANSLUZONE | 7-(2-methyl-2-propanyl)-2H-1,5-benzodioxepin-3(4H)-one |
| TRICYCLONE | (+)-(1R,7R)-10,10-DlMETHYL-TRICYCLO[7.1.1.0(2,7)]UNDEC-2-EN-4-ONE |
| VERDONE | (+-)-2-TERT-BUTYL-1-CYCLOHEXANON E |
| Z 11 CRUDE DIST | (1S,4S,9S,10R,13R)-5,5,9,13-tetramethyl-14,16-dioxatetracyclo[11.2.1.0~1,10~.0~4,9~]hexadeca ne (A) + (1R,4S,9S,10R,13S)-5,5,9,13-tetramethyl-14,16-dioxatetracyclo[11.2.1.0~1,10~.0~4,9~]hexadeca ne (B) |
| | (+-)-(4E,8E)-13-oxabicyclo[10.1.0]trideca-4,8-diene (A) + (+-)-(4E,8Z)-13-oxabicyclo[10.1.0]trideca-4,8-diene (B) + (+-)-(4Z,8E)-13-oxabicyclo[10.1.0]trideca-4,8-diene (C) |
| | MESO-(1R,2S,4R)-4-METHYL-TRICYCLO[5.2.1.0(2,6)]DECAN E-4-METHANOL |
| | (+-)-3-(3-METHYL-5-INDANYL)PROPANAL (A) + (+-)-3-(1-METHYL-5-INDANYL)PROPANAL (B) |
| | 2-METHYL-2-INDANMETHANOL |
| | (+-)-5-ETHYL-2-METHYL-2-INDANMETHANOL |
| | (+-)-5-ISOPROPYL-2-METHYL-2-INDANMETHANOL |
| | (2-METHYL-2-INDANYL)METHYL ACETATE |
| | (+-)-5-METHYL-2-INDANMETHANOL |
| | (+-)-(2,5-DIMETHYL-2-INDANYL)METHYL ACETATE |
| | 1-(2,5-DIMETHYL-2-INDANYL)-1-ETHANONE |
| | 1-(2,5-DIMETHYL-2-INDANYL)-1-ETHANOL |
| | 2-(2,5-DI M ETHYL-2-I N DANYL)-2-P ROPANOL |
| | 2-METHOXYMETHYL-2,5-DIMETHYLINDAN |
| | (+-)-PERHYDRO-2,5-DIMETHYL-CIS-2-INDENEMETHANOL |
| | (+-)-2-ETHYL-5-METHYL-2-INDANMETHANOL |
| | 2,5,6-TRIMETHYL-2-INDANMETHANOL |
| | (+-)-2,4-DIMETHYL-2-INDANMETHANOL |
| | (+-)-1,2,5-TRIMETHYL-2-INDANMETHANOL |
| | (+-)-1,2,6-TRIMETHYL-2-INDANMETHANOL |
| | (+-)-(2,4,5-trimethyl-2,3-dihydro-1H-inden-2-yl)methanol |
| | (+-)-2,3,4,5,6,7-HEXAHYDRO-2,5-DIMETHYL-2(1H)-INDENEMETHANOL |
| | (+-)-5-TERT-BUTYL-2-METHYL-2-INDANMETHANOL |
| | (+-)-(2,7-dimethyl-1,2,3,4-tetrahydro-2-napththalenyl)methanol |
| | (+-)-(2,6-dimethyl-1,2,3,4-tetrahydro-2-naphthalenyl)methanol |
| | (+-)-(5-METHOXY-2-METHYL-2,3-DIHYDRO-1H-INDEN-2-YL)METHANOL |
| | 2,4,6-TRIMETHYL-2-INDANMETHANOL |
| | (+-)-3-(3-ETHYL-2,3-DIHYDRO-1H-INDEN-4-YL)PROPANAL (A) AND/OR (+-)-3-(3-ETHYL-2,3-DIHYDRO-1H-INDEN-5-YL)PROPANAL (B) AND/OR (+-)-3-(1-ETHYL-2,3-DIHYDRO-1H-INDEN-5-YL)PROPANAL (C) AND/OR (+-)-3-(1-ETHYL-2,3-DIHYDRO-1H-INDEN-4-YL)PROPANAL (D) |
| | (4aRS,9bRS)-4a,8-dimethyl-4,4a,5,9b-tetrahydroindeno[1,2-d][1,3]dioxin |
| | (+-)-2-((methoxymethoxy)methyl)-2,5-dimethyl-2,3-dihydro-1H-indene |
| | (-)-(R)-2,5-DIMETHYL-2-INDANMETHANOL |
| | (4aRS,8aSR)-5,5,8a-trimethyloctahydro-2(1H)-naphthalenone |

**Table 2: Functional perfuming ingredients - Group II**

| Common Name | Chemical Name |
|---|---|
| METHYLCINNAMIC ALDEHYDE | (2E)-2-methyl-3-phenyl-2-propenal |
| CITRAL | (E)-3,7-DIMETHYL-2,6-OCTADIENAL (A) + (Z)-3,7-DIMETHYL-2,6-OCTADIENAL (B) |
| DAMASCONE ALPHA | (+-)-(2E)-1-(2,6,6-trimethyl-2-cyclohexen-1-yl)-2-buten-1-one |
| DAMASCONE BETA | (2E)-1-(2,6,6-trimethyl-1-cyclohexen-1-yl)-2-buten-1-one |
| DELTA DAMASCONE | (2E)-1-[(1RS,2SR)-2,6,6-trimethyl-3-cyclohexen-1-yl]-2-buten-1-one |
| GALIONE | (+-)-(E)-3-METHYL-4-(2,6,6-TRIMETHYL-2-CYCLOHEXEN-1-YL)-3-BUTEN-2-ONE (A) + (E)-1-(2,6,6-TRIMETHYL-2-CYCLOHEXEN-1-YL)-1-PENTEN-3-ONE (B) + (+-)-(E)-1-(2,2-DIMETHYL-6-METHYLENE-1-CYCLOHEXYL)-1-PENTEN-3-ONE (C) + (E)-1-(2,6,6-TRIMETHYL-1-CYCLOHEXEN-1-YL)-1-PENTEN-3-ONE (D) |
| GAMMA DAMASCONE | (+-)-(E)-1-(2,2-DIMETHYL-6-METHYLENE-1-CYCLOHEXYL)-2-BUTEN-1-ONE |
| IRONE ALPHA | (+-)-(E)-TRANS-alpha-IRONE (A) + (+-)-(E)-CIS-alpha-IRONE (B) + (+-)-(E)-beta-IRONE (C) |
| IRONE BETA | (+-)-(E)-4-(2,5,6,6-TETRAMETHYL-1-CYCLOHEXEN-1-YL)-3-BUTEN-2-ONE |
| ISORALDEINE 70 P | (+-)-(3E)-3-methyl-4-(2,6,6-trimethyl-2-cyclohexen-1-yl)-3-buten-2-one (A) + (+-)-(1E)-1-(2,6,6-trimethyl-2-cyclohexen-1-yl)-1-penten-3-one (B) |
| METHYLIONONE BETA | (1E)-1-(2,6,6-trimethyl-1-cyclohexen-1-yl)-1-penten-3-one |
| METHYLIONONE GAMMA | (+-)-(E)-3-METHYL-4-(2,6,6-TRIMETHYL-2-CYCLOHEXEN-1-YL)-3-BUTEN-2-ONE |
| ORIVONE | 4-(1,1-D!METHYLPROPYL)-1-CYCLOHEXANONE |
| PELARGODIENAL | (2 E,6Z)-2,6-NO NADI ENAL |
| VIOLETTE AT | (+-)-(3E)-4-(2,6,6-trimethyl-2-cyclohexen-1-yl)-3-buten-2-one (A) + (3E)-4-(2,6,6-trimethyl-1-cyclohexen-1-yl)-3-buten-2-one (B); |
| VIOLETTE AI | (+-)-(3E)-4-(2,6,6-trimethyl-2-cyclohexen-1-yl)-3-buten-2-one |
| VIOLETTE BC | (3E)-4-(2,6,6-trimethyl-1-cyclohexen-1-yl)-3-buten-2-one |
| ALDEHYDE C 11 UNDECYLIC | undecanal |
| CINNAMIC ALDEHYDE | (E)-3-PHENYL-2-PROPENAL |
| ALDEHYDE SUPRA | |
| ALPINOLIDE | (1S,1'R)-2-[1-(3',3'-DIMETHYL-1'-CYCLOHEXYL)ETHOXY]-2-METHYLPROPYL 2-PROPENOATE |
| ETHYL BUTYRATE | ETHYL BUTANOATE |
| CALONE | 7-methyl-2H-1,5-benzodioxepin-3(4H)-one |
| CARYOPHYLLENE | (-)-(1R,9S)-4,11,11-trimethyl-8-methylenebicyclo[7.2.0]undec-4-ene |
| CEDARWOOD OIL VIRGINIA | CEDARWOOD OIL VIRGINIA |
| CETOCEDRENE | 4,7,11,11-TETRAMETHYL-TRICYCLO[5.4.0.0(1,3)]UNDECAN-5-ONE (A) + 2,6,6,8-TETRAMETHYL-TRICYCLO[5.3.1.0(1,5)]UNDECAN-9-ONE (B) |
| CITRONELLOL BJ | (+-)-3,7-DIMETHYL-6-OCTEN-1-OL |
| CITRONELLYL NITRILE | (-)-(R)-3,7-DIMETHYL-6-OCTENENITRILE |
| COUMARINE | 2-CHROMENONE |
| DODECENAL | (2E)-2-dodecenal |
| FIRASCONE | methyl (1RS,2SR)-2,6,6-trimethyl-3-cyclohexene-1-carboxylate (A) + methyl (1RS,2RS)-2,6,6-trimethyl-3-cyclohexene-1-carboxylate (B) |
| LEMONILE | 3,7-DIMETHYL-2,6-NONADIENENITRILE (A) + 3,7-DIMETHYL-3,6-NONADIENENITRILE (B) |
| LILIAL | (+-)-2-methyl-3-[4-(2-methyl-2-propanyl)phenyl]propanal |
| LIMBANOL | (+-)-1-(2,2,3,6-TETRAMETHYL-CYCLOH EXYL)-3-HEXANOL |
| LIMINAL | (+)-(3S)-3-[(1R)-4-methyl-3-cyclohexen-1-yl]butanal (A) + (+)-(3R)-3-[(1R)-4-methyl-3-cyclohexen-1-yl]butanal |
| METHYLCITRAL | 3,6,7-TRIMETHYL-2,6-OCTADIENAL |
| PAMPLEWOOD | (+-)-3ENDO-METHOXY-7,7-DIMETHYL-10-METHYLENE-BICYCLO[4.3.1]DECANE (A) + (+-)-3EXO-METHOXY-7,7-DIMETHYL-10-METHYLENE-BICYCLO[4.3.1]DECANE (B) |
| TILLENAL | 3-(4,4-dimethyl-1-cyclohexen-1-yl)propanal |
| UNIPINE 85 | Alpha.-Terpineol + .Gamma.-Terpineol Mixture with other terpenes |
| VERTOXIME | 2-METHYL-3-HEXANONE OXIME |
| VIONIL 10 DIPG | (2Z,6Z)-2,6-nonadienenitrile (A) + (2E,6Z)-2,6-nonadienenitrile (B) |
| ZESTOVER | (1RS,2RS)-2,4-dimethyl-3-cyclohexene-1-carbaldehyde (A) + (1RS,2SR)-2,4-dimethyl-3-cyclohexene-1-carbaldehyde (B) |
| ALDEHYDE C 10 | DECANAL |
| ALDEHYDE C 12 | DODECANAL |
| ALDEHYDE C 8 | OCTANAL |
| ALDEHYDE C 9 | NONANAL |
| HEXYLCINNAMIC ALDEHYDE | (2E)-2-benzylideneoctanal |
| BASE XI | (+-)-5-heptyldihydro-2(3H)-furanone |
| BERGAMOT | |
| BERGAMOT ABERGAPT | BERGAMOT FU ROCOUMARIN-FREE |
| FIRWOOD | (+-)-(1-ethoxyethoxy)cyclododecane |
| LAVANDIN GROSSO ARR | |
| LAVANDIN GROSSO SYNTH | |
| METHYLOCTYNE CARBONATE (OCM) | METHYL 2-NONYNOATE |
| METHYLPARACRESOL | 1-METHOXY-4-METHYLBENZENE |
| TERPINOLENE | 1-methyl-4-(2-propanylidene)cyclohexene |
| UN DECAVERTOL | (+-)-(E)-4-METHYL-3-DECEN-5-OL |
| VIOLETTYNE 10 MIP | 1,3-UNDECADIEN-5-YNE |
| YLANG | |
| YLANG EXTRA | |

### Group I:

Ingredient(s) from Table 1 are comprised between 2 and 85wt% of the composition according to the present disclosure. According to one aspect, the composition used according to the present disclosure comprises a malodour antagonist system as defined above in an amount comprised between 6 and 70wt%. According to another aspect, the composition according to the present disclosure comprises a malodour antagonist system as defined above in an amount comprised between 8 and 60wt%. According to another aspect, the composition according to the present disclosure comprises a malodour antagonist system as defined above in an amount comprised between 8 and 46wt%.

According to a particular aspect of the present disclosure the malodour receptor antagonist system (group I) from the composition according to the present disclosure comprises at least 3 ingredients selected from Table 1. According to another aspect, at least 4, alternatively, at least 5, alternatively, at least 6, or alternatively, at least 8 ingredients selected from Table 1 are part of the malodour receptor antagonist system.

### Group II:

Group II in the present disclosure is a functional perfume accord as defined above. It is present in amounts ranging from 15 to 98wt% of the composition according to the present disclosure. According to one aspect, it is present in amounts ranging from 30-94wt%. According to another aspect, it is present in amounts ranging from 40-92wt% of the composition. According to another aspect, it is present in amounts ranging from 29-92wt% of the composition.

According to a particular aspect, group II consists of ingredients selected from the group consisting of ionones, irones, damascenes, citral, citronellol BJ, citronellyl nitrile, lemonile, methylcitral, cinnamic aldehyde, methylcinnamic aldehyde, hexylcinnamic aldehyde, pelargodienal, aldehyde C11 undecylic, aldehyde supra, dodecanal, aldehyde C8, aldehyde C9, aldehyde C12, orivone and mixtures thereof.

According to a particular aspect, group II consists of ingredients from the group of Table 2.

According to a particular aspect, group II consists of selected from the group consisting of ionones, irones, damascenes, citral, methylcinnamic aldehyde, pelargodienal, orivone, derivatives and mixtures thereof.

In some aspects, ionones, irones, damascones include damascone alpha, damascone beta, delta damascene, firascone, galione, gamma damascene, irone alpha, irone beta, isoraldeine 70 P, methyionone beta, methylionone gamma Coeur IFF, violet AI, violet AT, and violet BC.

In some aspects, methylcinnamic aldehyde includes alkyl derivatives, including cinnamic aldehyde, methylcinnamic aldehyde, hexythylcinnamic aldehyde.
In some aspects, methylcinnamic aldehyde includes alkyl derivatrives, including cinnamic aldehyde, methylcinnamic aldehyde, hexythylcinnamic aldehyde.

### Group III:

According to a particular aspect, the composition used according to the present disclosure comprise a nonfunctional perfume accord as defined above. The nonfunctional perfume accord consists of perfuming ingredients as defined above which are neither part of group II nor part of group I. If present in the composition according to the present disclosure, a non-functional perfume accord can typically be comprised in amounts ranging from 0.5 to 70wt%, alternatively, from 0.5 to 50wt% of the composition as defined in any of the above aspects.

### Group IV: delivery system

According to a particular aspect, compositions as defined above can be used in combination with a delivery system. The use of a delivery system allows achieving optimal gas-phase concentrations of active ingredients in the composition. Suitable delivery systems for the purpose of the present disclosure include but are not limited to:
∘ Passive plating supports comprising one or more of the following porous or non-porous substrates in loose powder or compacted form chosen from the following non-limiting examples: cellulose (paper/cardboard), vermiculite, other industrial absorbents, perlite, calcium carbonate, pumice, other minerals, wood, sawdust, ground corn cob, ground rice hull, rice hull ash, other agricultural by-products, biochars, starches, modified starches;
∘ Spray-dried moisture-activated encapsulation systems wherein compositions according to the present disclosure are encapsulated by a spray drying process within a matrix containing but not limited to one or more of the following: maltodextrin, octenyl succinated starch (modified starch);
∘ Core-shell encapsulation systems, such as mechanically activated microcapsules with an impermeable shell (for example, polyurea, polyurethane, and others) and composition according to the present disclosure in the core;
∘ Liquid mixtures containing surfactants;
∘ Polymeric materials.

Use of a composition as defined in any of the above aspects, wherein the composition further comprises encapsulating materials such as polymers to form microcapsules or microparticles, or materials to form liquid delivery system for the composition such as an emulsion, a microemulsion, a miniemulsion, a gel, a microgel, an anhydrous gel or a dispersion is therefore also an object of the present disclosure.

According to a particular aspect, the composition as defined in any of the above aspects is absorbed on a porous or non-porous substrate in loose powder or compacted form, the substrate being selected from cellulose (paper/cardboard), vermiculite, other industrial absorbents, perlite, calcium carbonate, pumice, wood, sawdust, ground corn cob, ground rice hull, rice hull ash, biochars, starches, modified starches and mixtures thereof.

According to a particular aspect, the composition comprises from about 6 to about 70wt% of group I. According to another aspect, the composition comprises from about 8 to about 60wt% of group I.

According to a particular aspect, the malodour receptor antagonist system comprises (2,5-dimethyl-2,3-dihydro-1H-inden-2-yl)methanol (LILYFLORE^{®}), in an amount of at least 2wt%, alternatively, at least 3wt% of the composition.

Without intending to be limited to any particular theory, combinations of ingredients within the malodour receptor antagonist system may exhibit a synergistic reduction or elimination of the perception of fecal malodour. Examples of such malodour counteracting compositions are shown in Example 16 below. Accordingly, in some aspects, the malodour receptor antagonist system comprises (2,5-dimethyl-2,3-dihydro-1H-inden-2-yl)methanol (LILYFLORE^{®}), and the functional perfume accord comprises isoraldeine (alpha-methylionone and isomethyl-alpha-ionone) and α-ionone (also referred to as Violet AT). Alternatively, the functional perfume accord further comprises citral.

Alternatively, in some aspects, the malodour receptor antagonist system comprises (2,5-dimethyl-2,3-dihydro-1H-inden-2-yl)methanol (LILYFLORE^{®}) and (+-)-3,7-dimethyl-1-octen-3-ol (dihydrolinalol), and the functional perfume accord comprises isoraldeine (alpha-methylionone and isomethyl-alpha-ionone), and α-ionone (also referred to as Violet AT).

The present disclosure's composition may be used in any consumer product for which it may be useful to have an MOC activity at least. Consequently, another object of the present disclosure is represented by a MOC consumer product comprising, as an active ingredient, at least one composition according to the present disclosure, as defined above.

The composition can be added as such or as part of a MOC composition (including a delivery system) according to the aspects presented herein.

It is understood that the MOC consumer product, by its nature can also be a perfuming one.

For the sake of clarity, it has to be mentioned that, by "MOC, and optionally perfuming, consumer product" or the similar, it is meant a consumer product which is expected to deliver at least a MOC effect, and optionally also a pleasant perfuming effect, to the surface to which it is applied (e.g. skin, hair, textile, or home surface, but also air). In other words, a consumer product according to the present disclosure is a perfumed consumer product which comprises the functional formulation, as well as optionally additional benefit agents, corresponding to the desired consumer product, e.g. a detergent or an air freshener, and an effective amount of at least one compound or composition from the present disclosure. For the sake of clarity, the consumer product is a non-edible product.

The nature and type of the constituents of the MOC consumer product do not warrant a more detailed description here, which in any case would not be exhaustive, the skilled person being able to select them on the basis of his general knowledge and according to the nature and the desired effect of the product.

Non-limiting examples of suitable perfuming consumer product can be:
- a fabric care product, such as a liquid detergent, a powder detergent, detergent tablets, a detergent bar, a detergent paste, a liquid fabric softener, fabric softener sheets, a fabric scent booster, a laundry pre-treatment, a fabric refresher, an ironing water, a laundry bleach, a carpet powder or a carpet cleaner; the uses for this type of product would be particularly beneficial in the cases where standard water available to the consumers could be associated with malodour as described (e.g. putrid waters);
- a toilet paper or napkin;
- an air freshening product, such as an air freshener spray, a gel air freshener, a liquid-wick air freshener, a solid air freshener comprising a porous substrate (such as a paper or card blotter, a porous ceramic, or a porous plastic), a liquid or gel air freshener comprising a permeable membrane, an electrically operated air freshener, and a dual purpose air freshener/disinfectant spray; and/or
- a surface care product, such as an all-purpose cleaner, a furniture polish, a wood floor cleaner, a toilet care product (such as a toilet bowl cleaning liquid, an in-cistern toilet cleaner, a toilet rim block, or a toilet rim liquid); a pet-litter.

Some of the above-mentioned MOC consumer products may represent an aggressive medium for the a compound according to some aspects of the present disclosure, so that it may be necessary to protect the latter from premature decomposition, for example by encapsulation or by chemically bounding it to another chemical which is suitable to release the ingredient upon a suitable external stimulus, such as an enzyme, light, heat or a change of pH.

The proportions in which the compound according to the present disclosure can be incorporated into the various aforementioned products or compositions vary within a wide range of values. These values are dependent on the nature of MOC consume product and on the desired organoleptic effect as well as the nature of the co-ingredients in a given composition when the compounds according to the present disclosure are mixed with other ingredients, solvents or additives commonly used in the art.

For example, in the case of perfuming compositions, typical concentrations are in the order of 0.01 % to 60 %, or even 1 % to 10 %, by weight, or even more, of the composition of the present disclosure based on the weight of the composition into which they are incorporated. Concentrations lower than these, such as in the order of 0.01% to 2% by weight, can be used when these compounds are incorporated into MOC consumer products, percentage being relative to the weight of the consumer product.

In particular, the concentration of MOC compound according to the present disclosure used in the various aforementioned consumer products varies within a various wide range of values depending on the nature of the consumer product.

### Examples

The present disclosure will now be described in further detail by way of the following examples, wherein the abbreviations have the usual meaning in the art and the temperatures are indicated in degrees centigrade (°C).

### Example 1

### Antagonist identification - Identification of malodour receptor antagonists through an ex vivo live neuron assay

In the *ex vivo* live neuron assay, olfactory sensory neurons (OSNs) are extracted from the olfactory epithelium of mice and can be tested for responses to sequentially delivered stimuli, where responses are detected through live-cell calcium-imaging microscopy. At least 1000 and approximately 5000-10000 OSNs were tested for every compound listed in table 1. It has been established through prior research in the field that the vast majority of extracted OSNs express 1 out of the approximately 1200 odourant receptors (ORs) present in the genome of a mouse, such that in our samples of extracted OSNs, the majority of the 1200 ORs should have been represented in at least one OSN. Since the responses of the OSNs to the delivered stimuli are entirely driven by the expressed OR, the OSNs selectively detect and collectively encode the identity and intensity of odourants. By stimulating the OSNs with MO molecules and measuring the response of each OSN, the subset in which a response is induced is those that detect and therefore presumably encode the MO. By subsequently delivering a mixture of MO and a candidate antagonist to the same cells, the degree of suppression of signal in each MO-responsive OSN can be determined ("level of inhibition"). The degree of inhibition in each cell was binned into one of three groups: low inhibition (10-25%), medium inhibition (25-75%) and strong inhibition (75-100%). In addition, the proportion of MO-responsive OSNs displaying low, medium and high inhibition was calculated. Examples of these data are shown in figures 1a-d. Compounds that inhibited greater than a minimum proportion of OSNs at a minimum strength were considered antagonist "hits" and putative malodour suppressing compounds. The minimum levels were, respectively, 10% of the population showing strong inhibition and/or 25% of the population showing medium inhibition and/or 40% of the population showing weak inhibition.

The genetic similarity between mouse and human receptors, due to their shared evolutionary history and presumably similar natural odour environments over evolutionary time-scales leads us to suppose that overall observations on MO-responsive populations of mouse ORs should positively correlate with what would be obtained from human ORs, even if individual orthologous receptors (i.e. those believed to share a common ancestor and typically the most similar in genetic sequence) may show varying levels of functional similarity to those from mouse.

Figures 1a-d show examples of results from live neuron assay antagonism screening against target fecal MOs, providing evidence of antagonism of MO receptors. Inhibition levels for the population of neurons were binned into high (75-100%, black), medium (25-75%, hashed) and low (10-25%, white). Compounds considered antagonists or "hits" were required to pass the population quantities denoted by the vertical lines (A, B, C) where the high inhibition must have passed A (10% of population of MO-responsive OSNs), and/or the medium inhibition must have passed B (25% of population of MO-responsive OSNs) and/or the low inhibition must have passed C (40% of population of MO-responsive OSNs).

### Example 2

### Sensory measurement of residual fecal odour score for individual malodour receptor antagonist system, individual functional perfuming ingredients and for compositions according to the present disclosure.

Malodour receptor antagonist system and compositions were submitted at a unique gas phase concentration of 3.4 µg/l air.

The sensory method to evaluate compositions requires the use of Firmenich designed air dilution olfactometers to achieve well controlled and stable gas phase concentrations of the compositions and of the malodour to a group of subjects.

The 30 subjects had to evaluate first the fecal reconstitution* alone and then rate the 3 attributes "Freshness", "Pleasantness" and "Fecal" (the malodour character) on a 0 to 10 scale. The next evaluation occurred 30 seconds later to avoid odour adaptation; the fecal malodour reconstitution was injected together with the tested composition in an olfactometer. Ratings for the same descriptors were recorded.
*The model malodour is a fecal reconstitution made of indole, methyl mercaptan, p-cresol and butyric acid. The gas phase concentration of the fecal malodour reconstitution and of its ingredients corresponds to the headspace analytical results from a toilet gas phase sampling (Charles JF Chappuis, Yvan Niclass, Christine Vuilleumier, and Christian Starkenmann Quantitative Headspace Analysis of Selected Odourants from Latrines in Africa and India Environ. Sci. Technol. 2015, 49, 6134-6140)

The results are expressed as the averaged rates for the three descriptors for the fecal reconstitution alone and the fecal reconstitution combined to the tested composition.

Figure 2 reports the results of score left when combining a malodour antagonist system consisting of (2,5-dimethyl-2,3-dihydro-1H-inden-2-yl)methanol (LILYFLORE^{®}), with a functional perfume accord consisting of α-ionone (also referred to as Violet AT) and isoraldeine.

It can be seen that a valuable depression of the perception of the fecal reconstitution (residual fecal odour < 50%) can be obtained when submitting at the same concentration:
- Independently a malodour antagonist system, in particular (2,5-dimethyl-2,3-dihydro-1H-inden-2-yl)methanol (LILYFLORE^{®}), and single ingredients of a functional perfume accord (Violet AT or isoraldeine), OR
- Combining the malodour antagonist system consisting of 2,5-dimethyl-2,3-dihydro-1H-inden-2-yl)methanol (LILYFLORE^{®}) with the functional perfume accord (under "mixture") OR
- Adding a malodour antagonist system with increasing numbers of antagonists from Table 1 to a composition comprising a functional and non-functional perfume accords.

Alias is a floral composition designed without including antagonists from Table 1 and including perfumery ingredients well known to those skilled in the art; however, it has a limited effect on the fecal reconstitution. The fecal score left when combining this composition to the fecal reconstitution is > 50%. This demonstrates that the malodour reduction effects are due to the antagonists are specific and not due to simple masking by perfumery ingredients.

### Example 3

### Sensory performance of compositions according to the present disclosure.

The capability of a mixture of a composition according to the present disclosure consisting of
- LILYFLORE^{®} ((2,5-dimethyl-2,3-dihydro-1H-inden-2-yl)methanol) as malodour antagonist system
- Isoraldeine and Violet AT (α-ionone) as functional perfume accord
to suppress a fecal model malodour* is significantly increased (70%) compared to the capability of each ingredient alone (max 58%) at its dosage in the mixture.

Illustration: Figure 3 gives a view of the 3 single compounds performance and of their mixture (a floral accord) against the fecal reconstitution.

Figure 3 in particular illustrates the capability of a composition according to the present disclosure to suppress a fecal model malodour. Each ingredient was tested alone at its dosage in the mixture. The mixture was tested at 3.4 µg/l air.

Blind sensory evaluations were organized; no information was disclosed to the 31 participants on the randomized submitted odourous stimuli. The test was duplicated and the observations accumulated.

### Example 4

### Compositions according to the present disclosure

Following tables represent compositions according to the present disclosure.

**Table 3 - Composition Floral E**

| Ingredients | Parts 1000 | |
|---|---|---|
| ALDEHYDE C 11 UNDECYLIC | 15 | FPI(functional Perfume Ing) |
| CITRONELLOL BJ | 200 | FPI(functional Perfume Ing) |
| HEXYLCINNAMIC ALDEHYDE | 150 | FPI(functional Perfume Ing) |
| ISORALDEINE 70 P | 70 | FPI(functional Perfume Ing) |
| LILIAL | 120 | FPI(functional Perfume Ing) |
| LILYFLORE^{®} | 100 | Antagonist System |
| PHENYLETHYL ACETATE | 240 | Antagonist System |
| VIOLET AT | 80 | FPI(functional Perfume Ing) |
| ZESTOVER | 25 | FPI(functional Perfume Ing) |

**Table 4 - Composition Floral P**

| Ingredients | Parts 1000 | Composition |
|---|---|---|
| ALDEHYDE C 11 UNDECYLIC | 15 | FPI(functional Perfume Ing) |
| CITRONELLOL BJ | 180 | FPI(functional Perfume Ing) |
| DAMASCONE ALPHA | 20 | FPI(functional Perfume Ing) |
| DELPHONE | 20 | Antagonist System |
| HEXYLCINNAMIC ALDEHYDE | 120 | FPI(functional Perfume Ing) |
| ISORALDEINE 70 P | 70 | FPI(functional Perfume Ing) |
| LILIAL | 120 | FPI(functional Perfume Ing) |
| LILYFLORE^{®} | 100 | Antagonist System |
| PHENYLETHYL ACETATE | 200 | Antagonist System |
| ROSINOL CRYST | 50 | Antagonist System |
| VIOLET AT | 80 | FPI(functional Perfume Ing) |
| ZESTOVER | 25 | FPI(functional Perfume Ing) |

**Table 5 - Composition Floral RD**

| Ingredients | Parts 1000 | Composition |
|---|---|---|
| ALDEHYDE C 11 UNDECYLIC | 15 | FPI(functional Perfume Ing) |
| CITRONELLOL BJ | 180 | FPI(functional Perfume Ing) |
| DAMASCONE ALPHA | 20 | FPI(functional Perfume Ing) |
| DELPHONE | 20 | Antagonist System |
| DIHYDROLINALOL | 130 | Antagonist System |
| HEXYLCINNAMIC ALDEHYDE | 120 | FPI(functional Perfume Ing) |
| ISORALDEINE 70 P | 90 | FPI(functional Perfume Ing) |
| LILYFLORE^{®} | 40 | Antagonist System |
| PHENYLETHYL ALCOHOL | 220 | Antagonist System |
| ROSINOL CRYST | 50 | Antagonist System |
| VIOLET AT | 90 | FPI(functional Perfume Ing) |
| ZESTOVER | 25 | FPI(functional Perfume Ing) |

**Table 6 - Composition Citrus B**

| Ingredients | Parts 1000 | |
|---|---|---|
| ALDEHYDE C8 | 30 | FPI(functional Perfume Ing) |
| ALDEHYDE C9 | 30 | FPI(functional Perfume Ing) |
| ALDEHYDE C10 | 50 | FPI(functional Perfume Ing) |
| ALDEHYDE C11 UNDECYLIC | 10 | FPI(functional Perfume Ing) |
| CITRAL | 170 | FPI(functional Perfume Ing) |
| CITRONELLOL BJ | 80 | FPI(functional Perfume Ing) |
| CITRONELLYL NITRILE | 120 | FPI(functional Perfume Ing) |
| ISORALDEINE | 100 | FPI(functional Perfume Ing) |
| LILYFLORE^{®} | 80 | Antagonist System |
| TERPINOLENE | 190 | FPI(functional Perfume Ing |
| VIOLET AT | 100 | FPI(functional Perfume Ing |
| ZESTOVER | 40 | FPI(functional Perfume Ing |

**Table 7 - Composition Citrus H**

| Ingredients | Parts 1000 | |
|---|---|---|
| ALDEHYDE C8 | 35 | FPI(functional Perfume Ing) |
| ALDEHYDEC9 | 30 | FPI(functional Perfume Ing) |
| ALDEHYDE C10 | 55 | FPI(functional Perfume Ing) |
| ALLYL AMYL GLYCOLATE | 3 | Antagonist System |
| BHT (IONOL) | 40 | NFPI (non functional perfume I) |
| CACHALOX^{®} | 2 | Antagonist System |
| CITRAL | 170 | FPI(functional Perfume Ing) |
| CITRONELLAL CP | 90 | Antagonist System |
| CITRONELLYL NITRILE | 100 | FPI(functional Perfume Ing) |
| CYCLOSAL | 90 | Antagonist System |
| DELTA DAMASCONE | 15 | FPI(functional Perfume Ing) |
| DIHYDROLINALOL | 100 | Antagonist System |
| ISORALDEINE | 45 | FPI(functional Perfume Ing) |
| LILYFLORE^{®} | 40 | Antagonist System |
| TERPINOLENE | 100 | FPI(functional Perfume Ing) |
| VIOLET AT | 45 | FPI(functional Perfume Ing) |
| ZESTOVER | 40 | FPI(functional Perfume Ing) |

**Table 8 - Composition Citrus 259389 B**

| Ingredients | Parts 1000 | |
|---|---|---|
| ALDEHYDE C8 | 35 | FPI(functional Perfume Ing) |
| ALDEHYDEC9 | 30 | FPI(functional Perfume Ing) |
| ALDEHYDE C10 | 55 | FPI(functional Perfume Ing) |
| ALLYL AMYL GLYCOLATE | 3 | Antagonist System |
| BHT (IONOL) | 40 | NFPI (non functional perfume I) |
| CACHALOX^{®} | 2 | Antagonist System |
| CITRAL | 180 | FPI(functional Perfume Ing) |
| CITRONELLAL CP | 100 | Antagonist System |
| CITRONELLYL NITRILE | 100 | FPI(functional Perfume Ing) |
| CYCLOSAL | 100 | Antagonist System |
| DELTA DAMASCONE | 15 | FPI(functional Perfume Ing) |
| DIHYDROLINALOL | 120 | Antagonist System |
| ISORALDEINE | 45 | FPI(functional Perfume Ing) |
| LILYFLORE^{®} | 40 | Antagonist System |
| TERPINOLENE | 100 | FPI(functional Perfume Ing) |
| VIOLET AT | 45 | FPI(functional Perfume Ing) |
| ZESTOVER | 40 | FPI(functional Perfume Ing) |

**Table 9 - Composition Jasmin E**

| Ingredients | Parts 1000 | |
|---|---|---|
| AMYL CINNAMIC ALDEHYDE | 75 | NFPI (non functional perfume I) |
| BENZYL ACETATE | 250 | Antagonist System |
| BENZYL PHENYLACETATE | 60 | NFPI (non functional perfume I) |
| CIS JASMONE | 30 | NFPI (non functional perfume I) |
| DECALACTONE CP | 25 | FPI(functional Perfume Ing) |
| DIHYDROLINALOL | 90 | Antagonist System |
| ETHYL 2 METHYLBUTYRATE @ 10% DIPG | 2 | NFPI (non functional perfume I) |
| ETHYL PRALINE | 7 | NFPI (non functional perfume I) |
| EUGENOL | 20 | NFPI (non functional perfume I) |
| ISOEUGENOL EXTRA NAT US | 4 | NFPI (non functional perfume I) |
| ISORALDEINE 70P | 100 | FPI(functional Perfume Ing) |
| LILYFLORE^{®} | 50 | Antagonist System |
| LINALYL ACETATE AR | 55 | NFPI (non functional perfume I) |
| METHYL ANTHRANILATE DIST | 2 | NFPI (non functional perfume I) |
| METHYL BENZOATE | 3 | FPI(functional Perfume Ing) |
| PARATOLYLALDEHYDE | 8 | NFPI (non functional perfume I) |
| PHENYLACETALDEHYDE | 4 | NFPI (non functional perfume I) |
| ROSINOL CRIST | 50 | Antagonist System |
| VIOLET AT | 150 | FPI(functional Perfume Ing) |
| ZESTOVER | 15 | FPI(functional Perfume Ing) |

### Example 5

### Sensory evaluation of compositions according to the present disclosure.

The sensory method to evaluate compositions described under example 4 required the use of Firmenich designed air dilution olfactometers to achieve well controlled and stable gas phase concentrations of the compositions and of the malodour to a group of subjects.

The 30 subjects had to evaluate first the fecal reconstitution alone and then rate the same descriptors as expressed previously on a line scale. The next evaluation occurred 30 seconds later to avoid odour adaptation; the fecal malodour reconstitution was injected together with the tested composition in an olfactometer. Ratings for the same descriptors were recorded.

The results are expressed as the averaged rates for the three descriptors for the fecal reconstitution alone and the fecal reconstitution combined to the tested composition.
Illustration: The graphs (Figure 4) represent the scores of Fecal, Freshness and Pleasantness attributes for the fecal reconstitution alone (unique concentration over the tests) and for the combination of the following compositions with this fecal reconstitution:
- A composition without identified antagonists (named Alias).
- Iterations of floral compositions (named Floral E, P and RD) gradually involving antagonist systems.
- Iterations of citrus compositions (named Citrus B and H) gradually involving antagonist systems.
- Iteration of Jasmin composition (named Jasmin E) involving antagonist systems.

The number and the % in weight of ingredients from Classes I (antagonist system), II (Functional perfume accord) and III (nonfunctional perfume accord) are indicated.

All these compositions are tested at 3 decreasing concentrations C1, C2 and C3.

The lower the fecal score, the more performing the antagonizing composition.
Figure 4a: Composition tested at 3.4 µg/lair (C1) against the fecal reconstitution
Figure 4b: Composition tested at 1.1 µg/lair (C2) against the fecal reconstitution
Figure 4c: Composition tested at 0.33 µg/lair (C3) against the fecal reconstitution

The dotted lines on the 3 graphs give the scores for the 3 attributes when evaluating the Floral RD, the Citrus H or the Jasmin E alone at C1 concentration (not combined to the fecal reconstitution). The three graphs indicate the minimum that may be expected for the Fecal score and the maximum scores for Freshness and Pleasantness.

The fecal score for Floral RD, Citrus H or Jasmin E evaluated alone at C1 concentration is not statistically different from the Fecal score of these compositions also tested at C1 concentration and combined to the fecal reconstitution (attested by Student's test, 99% confidence).
- The performance of the Floral and Citrus compositions improves when an antagonist system is added. From Floral E to Floral RD, the perception of the fecal malodour is increasingly reduced. A similar observation can be done for Citrus compositions with an increased performance of Citrus H versus Citrus B.
- The iterations gradually contain more ingredients in the antagonist system.
- The Jasmin composition also attests the interest in involving antagonist systems.
- The Floral RD, the Citrus H and the Jasmin E eliminate the fecal malodour perception.

### Example 6

Table 10 - "Floral V" is a floral-type fragrance composition according to the present disclosure, as follows:

| Ingredients | Parts 1000 | Composoition |
|---|---|---|
| ALDEHYDE C 11 UNDECYLIC | 17 | FPI(functional Perfume Ing) |
| BHT (IONOL) | 20 | NFPI (non functional perfume I) |
| CITRONELLOL BJ | 190 | FPI(functional Perfume Ing) |
| DAMASCONE ALPHA | 21 | FPI(functional Perfume Ing) |
| DELPHONE | 21 | Antagonist System |
| DIYDROLINALOL | 110 | Antagonist System |
| HEXYLCINNAMIC ALDEHYDE | 100 | FPI(functional Perfume Ing) |
| ISORALDEINE 70 P | 95 | FPI(functional Perfume Ing) |
| LILYFLORE^{®} | 40 | Antagonist System |
| PHENYLETHYL ALCOHOL | 232 | Antagonist System |
| ROSINOL CRYST | 52 | Antagonist System |
| VIOLET AT | 94 | FPI(functional Perfume Ing) |
| ZESTOVER | 8 | FPI(functional Perfume Ing) |

Table 11 "Citrus B2" is a citrus-like fragrance composition according to the present disclosure, as follows:

| Ingredients | Parts 1000 | |
|---|---|---|
| CITRAL | 180 | FPI(functional Perfume Ing) |
| DIHYDROLINALOL | 120 | FPI(functional Perfume Ing) |
| CITRONELLAL CP | 100 | FPI(functional Perfume Ing) |
| CITRONELLYL NITRILE | 100 | Antagonist System |
| CYCLOSAL | 100 | NFPI (non functional perfume I) |
| ALDEHYDE C 10 | 55 | Antagonist System |
| TERPINOLENE | 50 | FPI(functional Perfume Ing) |
| ISORALDEINE 70 P | 45 | Antagonist System |
| VIOLET AT | 45 | FPI(functional Perfume Ing) |
| BHT (IONOL) | 40 | Antagonist System |
| LILYFLORE^{®} | 40 | FPI(functional Perfume Ing) |
| ZESTOVER | 40 | FPI(functional Perfume Ing) |
| ALDEHYDE C 8 | 35 | Antagonist System |
| ALDEHYDE C 9 | 30 | Antagonist System |
| DELTA DAMASCONE | 15 | FPI(functional Perfume Ing) |
| ALLYL AMYL GLYCOLATE *α* | 3 | FPI(functional Perfume Ing) |
| CACHALOX^{®} | 2 | FPI(functional Perfume Ing) |

Table 12 "Jasmine E" is a jasmine-like fragrance composition according to the present disclosure, as follows:

| Ingredient | **Amount** (parts by weight) |
|---|---|
| BENZYL ACETATE *α* | 1250 |
| VIOLET AT *β* | 750 |
| ISORALDEINE 70 P *β* | 500 |
| DIHYDROLINALOL *α* | 450 |
| AMYLCINNAMIC ALDEHYDE R | 375 |
| BENZYL PHENYLACETATE *α* | 300 |
| LINALYL ACETATE AR | 275 |
| LILYFLORE^{®} *α* | 250 |
| ROSINOL CRYST *α* | 250 |
| CIS JASMONE | 150 |
| DECALACTONE CP | 125 |
| EUGENOL F | 100 |
| ZESTOVER | 75 |
| PARATOLYLALDEHYDE | 40 |
| ETHYL PRALINE | 35 |
| PHENYLACETALDEHYDE | 20 |
| ISOEUGENOL EXTRA NAT US | 20 |
| METHYL BENZOATE | 15 |
| METHYL ANTHRANILATE DIST | 10 |
| DIPROPYLENE GLYCOL | 9 |
| ETHYL 2 METHYLBUTYRATE | 1 |

### Example 7

### Latrine malodour reduction efficacy test of a cellulose-based air freshener comprising fragrance compositions according to the present disclosure.

The air freshener device used in this example was a cellulose air freshener-type. These air fresheners comprise of an absorbent material infused with a specified amount of fragrance. This material is then placed in a container to control the delivery of the fragrance composition. For this example, a cellulose pad is used as the absorbent material placed in an aluminum tin.

Test samples were prepared by applying 3 grams of fragrance compositions onto cellulose pads (2.5 in.²) that were placed in round aluminum tins (3 in. diameter). The fragrance compositions used for this test were "Floral V" (Example 6), two samples of "Citrus B2" (Example 6) and "Jasmine E" (Example 6).

A synthetic latrine malodour formulation was prepared as follows:

| Ingredient | w/w% |
|---|---|
| Triacetin | 99.755 |
| Indole | 0.1 |
| Butyric | |
| Acid | 0.009 |
| P-Cresol | 0.13 |
| DMTS | 0.006 |

A 70% by weight latrine malodour loaded vermiculite was prepared by admixing 350g of the latrine malodour with 150g of vermiculite (Fine grade, Specialty Vermiculite Corp, Enoree, SC).

The efficacy of the cellulose-based air fresheners comprising fragrance formulations according to the present disclosure was assessed following the practices described in ASTM E 1593-06 "Method for Assessing the Efficacy of Air Care Products in Reducing Sensorialy Perceived Indoor Air Malodour Intensity". Six 72ft³ (2.04m³) evaluation cabins with smelling windows within their doors were used for the sensory evaluation of samples. Five cabins contained a 3 inch (7.62cm) diameter aluminum tin with 9 grams of the latrine malodour loaded vermiculite; one cabin contained a 3 inch (7.62cm) diameter aluminum tin with 9 grams of vermiculite (without malodour)

One of the cabins containing the malodour only (no test product) was identified as a reference; the other five cabins were labeled with randomly generated 3 digit codes. The cabins set-up was as follows:

| Cabin Label | Cabin Contents |
|---|---|
| Reference | Latrine malodour only |
| 196 | Latrine malodour + Jasmine E |
| 274 | Latrine malodour + Citrus B2 |
| 326 | Latrine malodour only |
| 487 | Citrus B2 only |
| 571 | Latrine malodour + Floral V |

The cabins were assessed by 21 untrained but experienced assessors. By "untrained but experienced assessors" we mean individuals who have not received formal olfactive training but who are used to participating in fragrances assessments and have experience in rating the odour attributes.

The environmental conditions in the cabins during the test were 72°F (22°C), 35% RH with 5 air changes per hour. A portable desk fan, set on low, was placed at the floor of the cabin to circulate the air within. All assessors were first instructed to smell the odour in the reference cabin, in order to familiarize themselves with the malodour. They were then instructed to smell the odour in the test cabins and rate the intensity of the malodour using a 1 to 7 category scale, where 1 indicates no perceivable malodour and 7 indicates very strong malodour. Presentation of the test cabins was blind, balanced, randomized, and sequential monadic. Assessors were directed to open the smelling window to evaluate each sample and wait for 60 seconds before proceeding to the next.

Data was analyzed using one-way analysis of variance (ANOVA), followed by Fisher's least significant difference (LSD) method for multiple comparisons (α = 0.05). The number of assessors (N) and the LSD were as follows: N=21, LSD=0.60. Mean malodour intensity of the cabins is shown in on Figure 5.

The perceived malodour intensity of the Citrus B2 Only cabin (no malodour) is significantly lower than that of all other cabins. The perceived malodour intensity of the cabins containing malodour and fragrance compositions according to the present disclosure is significantly lower than that of the malodour only cabin; thus, cellulose air fresheners comprising fragrance compositions according to the present disclosure are useful in reducing the perception of latrine malodour.

### Example 8

### Latrine malodour reduction efficacy test of a candle comprising fragrance compositions according to the present disclosure.

The air freshener device used in this example was a candle; such devices deliver fragrance by two means. First, fragranced incorporated into the candle will evaporate slowly as it migrates through the wax and onto the surface of the candle. The second means, by far greater, is through the "melt pool". The melt pool is generated while the candle is lit and the flame melts portions of the candle forming a pool at the top. The warm mixture delivers fragrance at a greater rate.

The fragrance compositions used for this test were "Floral RD" (Example 4), two samples of "Citrus H" (Example 4) and "Jasmine E" (Example 6). The fragranced candles were prepared by mixing the aforementioned fragrance compositions with the candle formula indicated in the table below. 100 grams of the wax mixture was then placed in a 3 in. tall, round, glass container with a 3 in. diameter and a wax-coated, felt wick (CD# 6, clipped to a 0.5 in. height). For the test sample containing malodour only, a candle without fragrance was prepared (Candle wax 4625A IGI at 88%).

| Ingredient | Level |
|---|---|
| Candle wax 4625A IGI | 82.5% |
| Microwax 5715A IGI | 2.0% |
| Triple press Stearic acid | 10.0% |
| Fragrance | 5.5% |
| | 100.0% |

### Candle formulation

| Ingredient | Level |
|---|---|
| Candle wax 4625A IGI | 88.5% |
| Microwax 5715A IGI | 2.0% |
| Triple press Stearic acid | 10.0% |
| | 100.0% |

### Control formulation

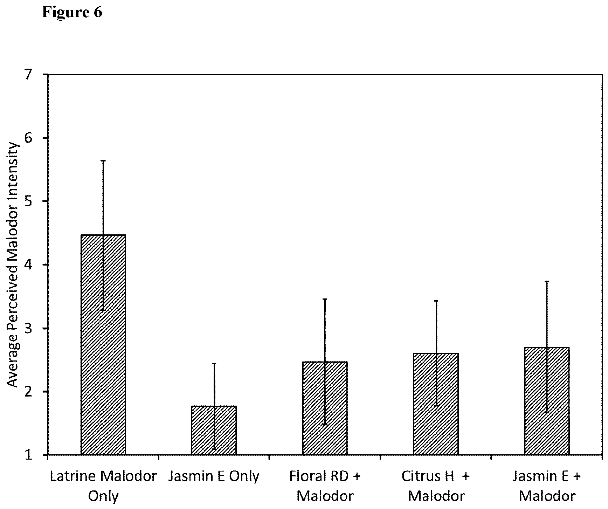

The malodour preparation and test procedure was the same as outlined in Example 6. The cabins were assessed by 15 untrained but experienced assessors. Data was analyzed using one-way analysis of variance (ANOVA), followed by Fisher's least significant difference (LSD) method for multiple comparisons (α = 0.05). The number of assessors (N) and the LSD were as follows: N=15, LSD=0.70. Mean malodour intensity of the cabins is shown in Figure 6.

The perceived malodour intensity of the Jasmine E Only cabin (no malodour) is significantly lower than that of all other cabins. The perceived malodour intensity of the cabins containing malodour and fragrance compositions according to the present disclosure is significantly lower than that of the malodour only cabin; thus, candles comprising fragrance compositions according to the present disclosure are useful in reducing the perception of latrine malodour.

### Example 9

### Latrine malodour reduction efficacy test of aerosol air fresheners comprising fragrance compositions according to the present disclosure.

The air freshener device used in this example was an aerosol; such devices deliver fragrance into an environment by means of a pressurized aqueous fragranced solution.

The fragrance compositions used for this test were "Floral V" (Example 6), two samples of "Citrus B2" (Example 6) and "Jasmine E" (Example 6). The fragranced aerosols were prepared by mixing fragrance compositions with the aerosol formula indicated in the table below.

| Ingredient | Level |
|---|---|
| Deionized Water | 69.25% |
| Sodium Borate | 0.07% |
| Sodium Molybdate | 0.34% |
| Span 80 | 0.25% |
| Dipropylene Glycol | 0.09% |
| Fragrance | 0.3% |
| A-60 Propellant | 29.7% |
| | 100.0% |

### Aerosol Formulation

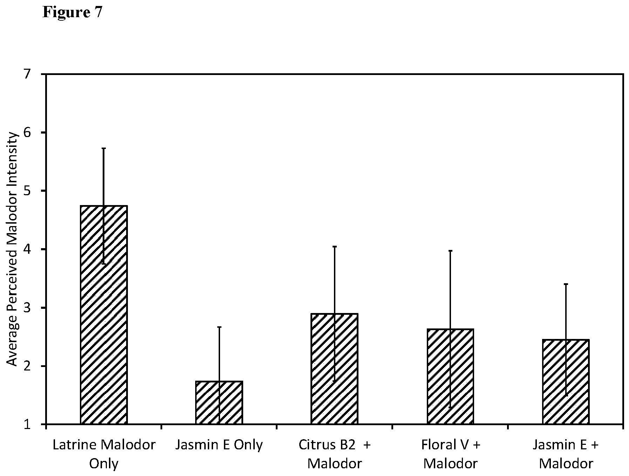

The malodour preparation and test procedure was the same as outlined in Example 6. The cabins were assessed by 19 untrained but experienced assessors. Data was analyzed using one-way analysis of variance (ANOVA), followed by Fisher's least significant difference (LSD) method for multiple comparisons (α = 0.05). The number of assessors (N) and the LSD were as follows: N=19, LSD=0.70. Mean malodour intensity of the cabins is shown in Figure 7.

The perceived malodour intensity of the Jasmine E Only cabin (no malodour) is significantly lower than that of all other cabins. The perceived malodour intensity of the cabins containing malodour and fragrance compositions according to the present disclosure is significantly lower than that of the malodour only cabin; thus, aerosol air fresheners comprising fragrance compositions according to the present disclosure are useful in reducing the perception of latrine malodour.

### Example 10

### Latrine malodour reduction efficacy test of sachet-type air fresheners comprising fragrance compositions according to the present disclosure.

The air freshener device used in this example was a sachet-type air freshener; such devices utilize a particulate substrate, infused with fragrance contained in a permeable pouch, the pouch being formed from paper, woven fabric or non-woven material.

The fragrance compositions used for this test were "Floral V" (Example 6), two samples of "Citrus B2" (Example 6) and "Jasmine E" (Example 6). The fragranced sachets were prepared by mixing the fragrance compositions with ground corn-cob particles (NatureZorb^{®} -100, origin: Aproa) at 20% loading by weight. 12 grams of the resulting mixtures were then placed in a 2.5 inch × 2.5 inch paper pouch. A sample comprising unfragranced corn-cob was prepared for the malodour only cabin.

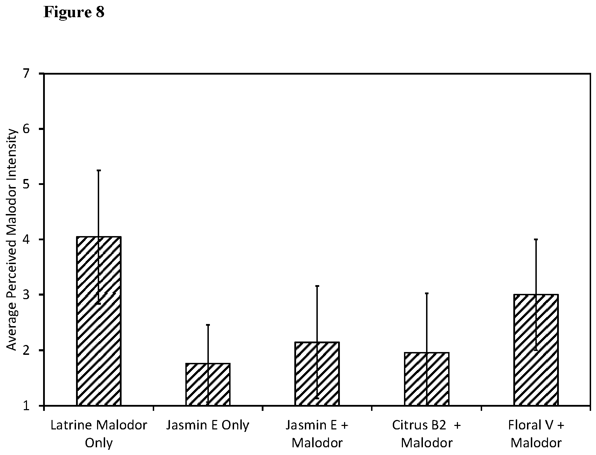

The malodour preparation and test procedure was the same as outlined in Example 8. The cabins were assessed by 21 untrained but experienced assessors. Data was analyzed using one-way analysis of variance (ANOVA), followed by Fisher's least significant difference (LSD) method for multiple comparisons (α = 0.05). The number of assessors (N) and the LSD were as follows: N=21, LSD=0.62. Mean malodour intensity of the cabins is shown in Figure 8.

The perceived malodour intensity of the Jasmine E Only cabin (no malodour) is significantly lower than that of the malodour only cabin and Floral V + malodour cabin. The Jasmin E + Malodour cabin and Citrus B2 + Malodour cabins were not perceived to be significantly stronger in malodour intensity than the cabin with no malodour, demonstrating how effective these two compositions are reducing the perception of latrine malodour. The perceived malodour intensity of the cabins containing malodour and fragrance compositions according to the present disclosure is significantly lower than that of the malodour only cabin; thus, sachet-type l air fresheners comprising fragrance compositions according to the present disclosure are useful in reducing the perception of latrine malodour.

### Example 11

### Latrine malodour reduction efficacy test of liquid electrical-type air fresheners comprising fragrance compositions according to the present disclosure.

The air freshener device used in this example was an electric-wick air freshener; such devices utilize a heating element to drive fragrance composition from a wick inserted into a reservoir with the fragrance.

The fragrance compositions used for this test were "Floral RD" (Example 4), two samples of "Citrus H" (Example 4) and "Jasmine E" (Example 6). The fragrance compositions were mixed with equal parts by weight Augeo Clean Multi (Solvay). 20 grams of the resulting mixtures were then placed in reservoirs with wicks (sintered plastic). The heater units used were designed to heat the wick to 70 °C

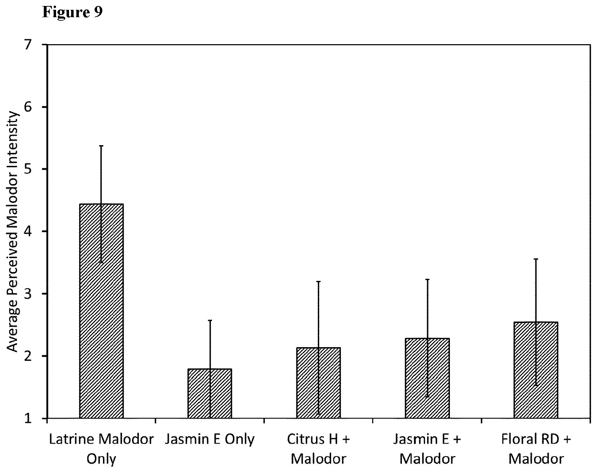

The malodour preparation and test procedure was similar to that outlined in Example 8. However, in this example the test cabins comprised a volume of 812 ft³ (23m³) and assessors assessed the odour by entering each cabin. Other details were as previously described. The cabins were assessed by 23 untrained but experienced assessors. Data was analyzed using one-way analysis of variance (ANOVA), followed by Fisher's least significant difference (LSD) method for multiple comparisons (α = 0.05). The number of assessors (N) and the LSD were as follows: N=23, LSD=0.56. Mean malodour intensity of the cabins is shown in Figure 9.

The perceived malodour intensity of the Jasmine E Only cabin (no malodour) is significantly lower than that of the malodour only cabin and Floral RD + malodour cabin. The Jasmin E + Malodour cabin and Citrus H + Malodour cabins were not perceived to be significantly stronger in malodour intensity than the cabin with no malodour, demonstrating how effective these two compositions are reducing the perception of latrine malodour. The perceived malodour intensity of the cabins containing malodour and fragrance compositions according to the present disclosure is significantly lower than that of the malodour only cabin; thus, liquid electrical-type air fresheners comprising fragrance compositions according to the present disclosure are useful in reducing the perception of latrine malodour.

### Example 12

The malodour reduction of fragrance composition described by the present disclosure was measured in a bleach cleaning powder.

The bleach cleaning powder is a bleach powder combined with spray-dried fragrance. Standard usage of this product is to apply the powder to the area to be treated and dissolved with water, followed by scrubbing to loosen all particles and then rinsed.

Fragranced bleach samples were prepared by adding 0.15 grams of spray-dryed powder (comprised of 50% w/w perfume, 50% w/w octenyl succinated modified starch) to 9.85 grams of Stable Bleaching Powder (Grade I, Gujarat Alkalies and Chemicals Limited, Gujarat, India).

The malodour composition detailed in Table 1 was applied onto fine vermiculite (Specialty Vermiculite Corp, Enoree, SC) with a 70% loading by weight. 9 grams of the composition was then presented to assessors in round aluminum tins. The aluminum tins have a 3 in. diameter with a 1 in. height. For the test sample with fragrance only, a tin with untreated vermiculite is used.

**Table 13**

| Ingredient | w/w% |
|---|---|
| Triacetin | 99.8775 |
| Indole | 0.0500 |
| Butyric | |
| Acid | 0.0045 |
| P-Cresol | 0.0650 |
| DMTS | 0.0030 |

A 70% by weight latrine malodour loaded vermiculite was prepared by admixing 350g of the latrine malodour with 150g of vermiculite (Fine grade, Specialty Vermiculite Corp, Enoree, SC). The efficacy of the cellulose-based air fresheners comprising fragrance formulations according to the present disclosure was assessed following the practices described in ASTM E 1593-06 "Method for Assessing the Efficacy of Air Care Products in Reducing Sensorialy Perceived Indoor Air Malodour Intensity". A booth labeled "Reference" containing only a malodour tin was presented to assessors first to familiarize them with the malodour. Using a scale of 1 to 7 (1 signifying no odour, 4 moderate odour and 7 extremely strong malodour), assessors were then asked to evaluate each sample in specified order and rate malodour intensity and total odour intensity.

The samples were assessed by 19 untrained assessors. By "untrained assessors" we mean users of air fresheners who have not received formal olfactive training but who are used to participating in fragrances assessments and have experience in rating the odour attributes.

Using 60 ft³ (1.7m³⁾ evaluation rooms, the floors of the cabins were wet with water and samples were scrubbed onto the floors until dissolved. The malodours were then added. The environmental conditions during the test were 72 F, 40% RH with 5 air changes per hour. A portable desk fan, set on low, is placed at the floor of the cabin to circulate the air within. Booths were labeled with a randomly generated 3 digit code. Sample presentation was blind, balanced, randomized and sequential monadic. After 5 minutes from activation, assessors were directed to open the smelling window to evaluate each sample and wait for 60 seconds before proceeding to answer a series of questions relating to the odour they perceived in the room. The assessors were asked to rate the malodour strength and total odour intensity.

Data was analyzed using Analysis of Variance (ANOVA) and the difference between two means determined using the least significant difference (α = 0.05). Mean malodour intensity of the cabins is shown below; the least significant difference between means was 0.61.

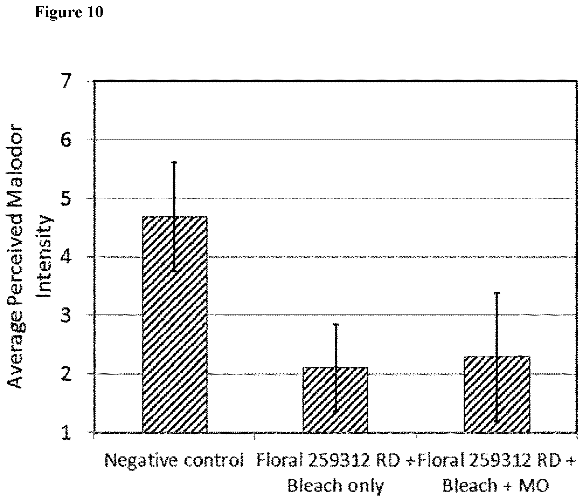

### Results are shown in Figure 10

The perceived malodour intensity of the Floral RD + Bleach Only cabin (no malodour) is significantly lower than that of the malodour only cabin. The Floral RD + Bleach + Malodour cabin was not perceived to be significantly stronger in malodour intensity than the cabin with no malodour, demonstrating how effective this composition is at reducing the perception of latrine malodour. The perceived malodour intensity of the cabin containing malodour and Floral RD composition according to the present disclosure is significantly lower than that of the malodour only cabin; thus, bleach cleaning powders comprising fragrance compositions according to the present disclosure are useful in reducing the perception of latrine malodour.

### Example 13

### Latrine malodour reduction efficacy test of fragrance compositions according to the present disclosure in a model latrine

In this example, in order to evaluate performance of compositions according to according to the present disclosure, a model latrine was constructed. The model latrines were equipped with an odour generator that injected hydrogen sulfide, methyl mercaptan, butyric acid, para-cresol, and indole, allowing the accurate and reliable reconstitution of a toilet malodour headspace. The malodourant concentrations in the model latrines matched the quantitative headspace analysis made in African and Indian toilets. The toilet malodour headspace performances were validated by chemical and sensory analysis. Olfactory stimuli were presented to participants in different climates to assess the effect of climate on the perception of odours. The sensory data showed that increasing temperature and humidity decreased the intensity ratings of malodours but not their quality. Perfume formulations can be delivered in these model latrines by forced evaporation to control the headspace concentration or by delivery systems such as cellulosic pads, liquids, and powders. Our experimental setup provided dose-response curves to assess the performance of perfume formulations in reducing toilet malodour and increasing pleasantness.

### Material and Methods

### Chemicals

The compounds triethylamine, N-ethylmaleimide (NEM), and methyl octanoate were purchased from Sigma-Aldrich (Buchs, Switzerland), and butyric acid, p-cresol, indole, and L-cysteine were in-house products. The solvents diethyl ether, methanol, ethyl acetate, and acetone were purchased from Carlo Erba (Val de Reuil, France). For methyl mercaptan and hydrogen sulfide, nitrogen mixtures at 15 ppm (v/v) were used, in pressurized cylinders purchased from Carbagas (Carouge, Switzerland). Oasis HLB 1-g cartridges were purchased from Waters (Montreux-Chailly, Switzerland). The perfume formulation used in this example is described below.

**Table 14: Floral D Perfume Formulation**

| Molecule Name | Parts |
|---|---|
| UNDECANAL | 15 |
| (E)-2-PENTYL-3 -PHENYL-2-PROPENAL | 150 |
| (+-)-3,7-DIMETHYL-6-OCTEN-1-OL | 200 |
| (+-)-(3E)-3-methyl-4-(2,6,6-trimethyl-2-cyclohexen-1-yl)-3-buten-2-one (A) + (+-)-(1E)-1-(2,6,6-trimethyl-2-cyclohexen-1-yl)-1-penten-3-one (B) | 70 |
| (+-)-2-methyl-3-[4-(2-methyl-2-propanyl)phenyl]propanal | 120 |
| (+-)-2,5-DIMETHYL-2-INDANMETHANOL | 100 |
| 2-PHENYLETHANOL | 240 |
| (+-)-(3E)-4-(2,6,6-trimethyl-2-cyclohexen-1-yl)-3-buten-2-one (A) + (3E)-4-(2,6,6-trimethyl-1-cyclohexen-1-yl)-3-buten-2-one (B); | 80 |
| (1RS,2RS)-2,4-dimethyl-3-cyclohexene-1-carbaldehyde (A) + (1RS,2SR)-2,4-dimethyl-3-cyclohexene-1-carbaldehyde (B) | 25 |

### Model latrines

Three 1.7 m³ model latrines (1.95 m × 0.985 m × 0.89 m) made of 8-mm transparent polyethylene terephthalate were placed in a climate chamber, and each latrine was equipped with a 29 cm × 39 cm rotating door to allow evaluation of odours (Figure 11). The air from the climate chamber entered each latrine via the odour generator placed at a height of 45 cm in the back wall (the odour generator is described in detail below). The air was sucked from the roof of each latrine through a double-sided 82 cm × 91 cm laminar filter (thick cotton fabric) via a 100-mm aluminium exhaust tube (Figure 11). The three exhaust tubes were connected to an adjustable fan via a main 100-mm stainless steel pipe. The airflow of each latrine could be separately adjusted with a damper (SPI 160, Systemair, Skinnskatteberg, Sweden) placed in the exhaust pipe (Figure 11). A hot wire anemometer was placed in the main exhaust pipe to control the main exhaust flow. The air flow could be adjusted by changing the suction force produced by the fan if necessary. The air velocity inside the main exhaust pipe was maintained at an airflow of 17 m³/h in each latrine. This airflow and the resulting air changes per hour (roughly 10) are in the range of measurements made in a ventilated improved pit latrine.

### Odour generator

To force the evaporation of liquids, we modified the lower chamber of a publically available olfactometer. A 150 L/h nitrogen flow flushed a 500-ml round-bottom flask, where liquids were introduced via a polytetrafluorethylene (PTFE) capillary linked to a 1-ml polypropylene syringe (Figure 11). The flow rate of liquids was delivered by a syringe pump and could range from 0.04 ml/h to 10 ml/h. The flask was covered with a glass hat and heated to 160°C with a stainless steel heat-on block mounted on a heating plate (Figure 11). The outlet of the flask (2 mm inner diameter) was placed in a 15-cm-long stainless steel pipe of 109 mm in diameter that crossed the back wall of the latrine. This pipe was connected to a 127-mm diameter aluminium exhaust pipe (80 cm long) placed in the back wall (Figure 11) to avoid sucking heated air inside the latrine. The nitrogen that was enriched with odourant molecules mixed with the air that entered the latrine. In addition to the forced evaporation release systems, two stainless steel tubes were soldered inside the pipe to hold 6-mm PTFE tubes to release methanethiol and hydrogen sulfide from pressurized cylinders. The PTFE tubes were closed with Parafilm and two 0.8-mm openings per tube were pierced with a needle (Figure 11).

The nitrogen flow rate of each latrine was controlled with three rotameters (25 L/h to 250 L/h; Krohne, Duisburg, Germany). The flow rates of methanethiol and hydrogen sulfide were controlled independently with six mass flow meters (three toilets, two gases) (Red-y, Vögtlin Instruments AG, Aesch, Switzerland).

### Climate chamber

The climate chamber dimensions were 3.42 m × 2.95 m × 2.5 m, resulting in a volume of 25 m³. The temperature and humidity of the climate chamber was controlled in a closed cycle of 540 m³/h. Fresh air entered the chamber at a rate of 51 m³/h and air left the chamber at the same rate. The working range for temperature and relative humidity (RH) was 12 °C to 45 °C and 30% RH to 90% RH, respectively. The climate chamber was equipped with temperature and RH probes placed at the entrance and outlet of the temperature and humidity controlling cycle. The data from the probes at the entrance were recorded every 5 min, allowing the measurement of temperature and RH of the air inside the climate chamber during the experiments. Moreover, a proble was placed (Traceable^{®} hygrometer, VWR International, Radnor, PA, USA) inside the latrine to punctually measure the RH and temperature to ensure that the differences in temperature and RH between the air inside the climate chamber and the air inside the latrines was minimal. A temperature difference below 1.5 °C and a RH difference below 5% was mainteind.

### Participants

The participants were employees from the research center at Firmenich SA (Geneva, Switzerland). Ten sessions were organized and the number of participants for each session was as follows: 26, 24, 26, 27, 26, 30, 25, 27, 25, 23. The participants signed a consent form before participating in the study. The consent form and experimental protocol were approved by the internal review board of Firmenich in agreement with the Declaration of Helsinki for medical research involving human subjects.

### Stimuli

The participants were exposed to six odourant mixtures delivered in latrines in four different climates. The odourant mixtures were Mukuru (Nairobi) UDT malodour alone, the perfume (Floral D) alone, and mixtures of the malodour and the perfume released at four different concentrations (0.18, 0.54, 1.62, 4.9 µg/l).

The malodour was reconstituted from Mukuru toilets because it contains all of the significant molecules and it came from well-maintained toilets. The Mukuru malodour source was composed of hydrogen sulfide, methanethiol, butyric acid, p-cresol, and indole, whose gas phase concentrations were 0.26, 0.018, 0.004, 0.0027 and 0.00018 µg/l, respectively. Hydrogen sulfide and methanethiol were released from pressurized cylinders at 20.8 l/h and 9.8 l/h, respectively. The remaining malodour products were released in the latrines by forcing the evaporation of a propylene glycol solution that contained 0.775, 0.526 and 0.035 mg/ml of butyric acid, p-cresol and indole, respectively. The perfume formulation was released in pure form in the forced evaporation chamber, resulting in a gas phase concentration of 4.9 µg/l. The lower gas phase concentration of the perfume was achieved by diluting it in propylene glycol. The gas phase concentrations of 0.18, 0.54 and 1.62 µg/l were obtained with 3.62%, 11.13% and 33.31% (w/w) propylene glycol solutions, respectively. To release the malodour and the perfume in the same latrine, two syringes via two PTFE capillaries were connected to the same forced evaporation chamber. One syringe contained the malodour solution and the other syringe contained the perfume formulation, either in pure form or diluted in propylene glycol. Both syringes were mounted on the same syringe pump and their pistons were pushed at 0.088 mm/h, resulting in a release rate of 0.088 ml/h. When the malodour or the perfume was presented alone, pure propylene glycol was injected into the forced evaporation chamber with the second syringe. Each odour was presented in four climates: 22°C at 30% RH, 22°C at 80% RH, 35°C at 30% RH, and 35°C at 80% RH.

### Sensory protocol

The participants were randomly exposed to the odour stimuli in the different climates. As only three latrines were available, the six odours were split into two groups, each containing the malodour alone or the perfume alone, the malodour plus a low dose of perfume, and the malodour plus a high dose of perfume. The first and the last sessions were used as controls to assess the reliability of the panel and were composed of the malodour alone, the perfume alone, and a mixture of both. The participants entered the climate chamber and directly evaluated the odour of the three latrines by answering a paper questionnaire made with the software FIZZ (Biosystems, Courtenon, France). They reevaluated the odour of each latrine after a 3-min adaptation to the climate. They were asked to rate, on 0-10 linear scales, the pleasantness from "I don't like" to "I like," the familiarity from "not familiar" to "very familiar," the intensity from "no odour" to "very strong," the fecal/toilet character from "not fecal/toilet" to "very fecal/toilet," and whether they wanted to enter the latrine from "not at all" to "very willingly."

### Headspace analysis

The Mukuru malodour was released in the model latrines as described above. The climate was set to 25 °C at 50% RH. The compounds released into the air were collected with Oasis cartridges conditioned with 20 ml of deionized water, 20 ml of methanol, 20 ml of acetone, and 20 ml of diethyl ether and dried at 50 °C for 1 h in an oven. Hydrogen sulfide and methyl mercaptan were derivatized with NEM in Oasis cartridges loaded with 2 ml of diethyl ether containing 25 mg NEM and 100 µl of triethylamine and dried for 1 h at 50 °C. The air was pumped at 1 l/min through the cartridges by using GilAir Plus pumps connected with silicon tubes. The volume of the samples was 1001. Three cartridges were used to sample the air of one latrine. One cartridge was placed in the center of the model latrines, the second 23 cm from the evaluation door, and the third deep at the top right of the latrine (171 cm from the ground). The cartridges were desorbed with 10 ml of diethyl ether added to 100 µl of 10 ng/µl methyl octanoate (internal standard [IS]) solution in ethyl acetate. To remove the excess NEM, the eluate was washed with 3 ml of 10 mg/ml of L-cysteine solution in water buffered at pH 8 with 0.1 M potassium phosphate. The water phase was removed and the organic phase dried with sodium sulfate. The water phase was acidified with 100 µl of a 37% HCl solution in water; butyric acid was extracted with 4 ml of diethyl ether added to 100 µl of IS. Prior to injection in the GC-MS, both organic phases were gently concentrated to 1 ml under argon flow. The analysis was performed by injecting 1 µl of the eluate into the GC-MS as described below.

### Headspace analysis calibration using an olfactometer

Using an olfactometer, a headspace was created with known concentrations of butyric acid, indole, p-cresol, methyl mercaptan, and hydrogen sulfide to calibrate the analytical method. Briefly, air with known amounts of compounds was sampled with Oasis cartridges loaded with the derivatization agent NEM (described above) at the outlet of the olfactometer. Methyl mercaptan and hydrogen sulfide were released into the olfactometer from pressurized cylinders containing a mixture of 15 ppm of both sulfur compounds in nitrogen. The flow of both sulfur compounds was controlled with rotameters (Vögtlin TV 100). Butyric acid, indole, and p-cresol were released by forcing the evaporation of propylene glycol solutions from a 1-ml polypropylene syringe mounted on a syringe pump delivering 0.101 ml/h. The solution was introduced into the lower chamber, which was heated to 150 °C by using an oil bath. Nitrogen was introduced into the lower chamber at 60 l/h to collect the products that evaporated and was mixed with the airflow of the upper chamber. The airflow was set at 540 l/h and humidified by bubbling in a water-jacketed wash bottle filled with distilled water. The upper chamber of the olfactometers was water-jacketed and its temperature was maintained at 29 °C with a water bath. At the outlet of the olfactometers, the temperature was 30 °C and the RH was 40%. The resulting concentrations of methyl mercaptan and hydrogen sulfide compounds in the olfactometer were 0.1, 0.05, 0.0250 and 0.0125 µg/l. The resulting concentrations of butyric acid, p- cresol, and indole were 0.0001, 0.001, 0.01 and 0.1 µg/l.

### Gas chromatography-mass spectrometry (GC-MS)

A GC 6890 N (Agilent, Palo Alto, CA, USA) was used to identify the compounds. A fused silica SPB-1 capillary column (30 m × 0.25 mm i.d., 0.25-µm film thickness, Supelco, Bellefonte, PA, USA) was mounted in the GC. The carrier gas was He (52 kPa) and the injector temperature was set at 250 °C. Injections were made with a Combi-Pal autosampler (Zwingen, Switzerland). To analyze butyric acid, p-cresol, indole, and NEM derivatives of methyl mercaptan and hydrogen sulfide, the initial oven temperature was held at 50°C for 5 min and then increased at 5°C/min to 250 °C, split mode 1/5. The GC was coupled to a MS 5975B Inert XL MSP from Agilent. The mass spectra in the electron impact mode were measured at 70 eV in SIM mode. The ions that were monitored were butyric acid (60), p-cresol (107), indole (117), NEM-S-CH₃ (127), and NEM-S-NEM (127).

### Data analysis

The questionnaires were scanned and the data stored in FIZZ and analyzed with R (https://cran.r-project.org). The response variables-pleasantness, enter the latrine, intensity, familiarity, and fecal character-were analyzed by using analysis of variance (ANOVA), and any significant effect was confirmed with the non-parametric Kruskal-Wallis test. Pairwise comparison tests were made with the Tukey honest difference test (Tukey HSD function in R). The relationship between the pleasantness of the different odour treatments and the willingness to enter the latrines was investigated with linear models. Moreover, pleasantness ratings from odour treatments and pleasantness ratings from the climates were analyzed with linear models. The level of significance was set at P < 0.05. To determine the concentrations of malodour compounds in the gas phase of the model latrines, calibration curves were established by using linear models on the ratio of the peak area of volatiles and IS as a function of the gas phase concentrations in the olfactometer. Using these calibration curves and the inverse prediction function in R (*chemCal* package), the gas phase concentrations inside the model latrines were predicted from the ratios of peak area of volatiles and IS.

### Results

Three toilet models of 1.7 m³ were built in a climate chamber of 25 m³. Each toilet had 10 air changes per hour. Inside the toilets, a scale was installed to monitor weight gain or loss of and hard surfaces to receive liquids or powders. When the subjects stepped into the climate chamber, they were exposed to the temperature and humidity set up for the experiment; therefore, for the present study, they were asked to make a first evaluation of the odour directly after entering the chamber and to make a second evaluation after a few minutes of adaptation to the climate. Adaptation had no significant effect on the criteria used to evaluate the odour. The data with and without adaptation were then averaged.
A typical Mukuru fecal toilet malodour was created through a controlled release of methanethiol, hydrogen sulfide, butyric acid, p-cresol, and indole by spraying the gas and vaporizing the liquids in a hot chamber flushed with nitrogen (Figure 11). The headspace was analyzed in three different locations in the toilets and in the three toilets. The results of the quantifications, compared with the expected concentrations, are shown in Figure 12. The target concentrations of methanethiol, hydrogen sulfide, butyric acid, p-cresol, and indole were attained at 101%, 66%, 130%, 93%, and 138%, respectively, of the expected values. The standard deviations showed a relatively small interval, indicating homogeneous headspace inside the cabins in addition to a reproducible headspace between the cabins.

Four descriptors were proposed to the subjects: pleasantness, enter the toilet, fecal character and intensity. The panel was reliable, as the results obtained with the panel when we repeated the first and last sessions are not significantly different (Figure 13).

The climate significantly affected the intensity, but had no significant effect on the other criteria of pleasant, familiarity, fecal character, and willingness to enter the latrines. The increase in temperature significantly decreased the overall intensity (ANOVA, P < 0.0001; Kruskal-Wallis, P < 0.001) independently of the odour (Figure 14). Similarly, but to a lesser extent, an increase in humidity significantly decreased the intensity (ANOVA, P < 0.05; Kruskal-Wallis, P < 0.05), as shown in Figure 14. However, an increase in both humidity and temperature did not significantly combine to further depress the overall intensity. The significant effect of temperature was mainly due to differences in means obtained with the malodour alone, the mixtures of the malodour and the highest perfume concentration, and the perfume alone. The significant effect of humidity was mainly due to the mixtures of the malodour and the highest perfume concentration and to the perfume alone. The odours had no significant effect on the intensity, as pairwise comparisons (Tukey honest difference) revealed no significant differences (Figure 14).

Next, the appropriate control was evaluated. The choices were: the malodour (blue bars, Figure 15 and 16) or the perfume (pink bars, Figure 15 and 16). The fecal character was significantly depressed when the perfume concentration was increased, as shown in Figure 15. The reduction in this character as a percentage of the fecal character of the malodour is also shown in Figure 15. However, the efficiency of the perfume was reduced when the mixtures of malodours and perfume were presented with the perfume alone (blue bars, Figure 15). In contrast, the efficiency of the perfume was higher when the mixtures were presented with malodour alone (pink bars, Figure 15).

As opposed to the direction of the fecal character ratings, the pleasantness ratings increased significantly as a function of increasing concentrations of perfume (Figure 16). The treatment groups also had an effect on pleasantness, but only with the lower perfume concentrations (Figure 16).

A similar result was obtained with the ratings of willingness to enter the latrines compared with the pleasantness ratings. Ratings of willingness increased significantly as a function of increasing perfume concentrations. The willingness to enter the latrine was strongly correlated to those of pleasantness (Figure 17, linear model, slope = 0.97, P < 0.0001; intercept = 0.27, P < 0.001, adjusted R² = 0.7986). This linear model explained roughly 80% of the variance of the enter ratings with the pleasantness on the abscissa. The effect of the treatment groups on the enter ratings was much lower than that on the pleasantness ratings.

### Temperature measurements

The climate chamber was set for four climate conditions: 22°C at 30% RH, 22°C at 80% RH, 35°C at 30% RH, and 35°C at 80% RH. The temperature and RH conditions were reached by using the temperature controlling system (Table 15). The temperature and the RH inside the climate chamber and inside the model latrines were less than 1.5 °C and 5%, respectively (Table 15).

**Table 15. Measurements of temperature and relative humidity inside the climate chamber and inside the latrine**

| Temperatur e set (°C) | RH set (%) | Average temperature of climate chamber [°C] | Standard deviation of temperatur e of climate chamber | Average RH of climate chamber [%] | Standard deviation of RH of climate chamber | Temperatur e of latrine | RH of latrine |
|---|---|---|---|---|---|---|---|
| 22 | 30 | 22.0 | 0.6 | 32.6 | 3.1 | 23.0 | 31.3 |
| 35 | 30 | 35.0 | 0.2 | 30.4 | 0.9 | 34.4 | 28 |
| 22 | 80 | 22.0 | 0.2 | 73.0 | 2.5 | 22.8 | 75.5 |
| 35 | 80 | 35.0 | 0.2 | 76.0 | 2.2 | 34.8 | 81 |

### Example 14

### Latrine malodour reduction efficacy test of fragrance compositions according to the present disclosure in latrines using passive delivery systems

Without intending to be limited to any particular theory, the performance of the compositions may be influenced by factors, such as, for example, the volatility of the compound in the formulation, the temperature, airflow, the depth of the boundary layer, the interactions of the compounds with the substrate of the passive delivery system, the interactions between the compounds, the concentrations of each compound in the delivery system, climate, and the like. Such factors may influence the influence the duration and/or the magnitude of the perceived reduction in fecal malodour, and/or the duration and/or the magnitude of changes in other sensory effects, such as, for example, an increased in perceived pleasantness.

To explore this further, in this example, the performances of compositions according to some aspects of the present invention were evaluated in latrines, where the compositions were incorporated into passive delivery systems. In a first series of experiments using a model system, the following two formulations were tested: Floral V (as described in Table 10), and Jasmin E (as described in Table 9). A panel of 19 to 32 participants was trained on-site to evaluate the performance of the test compositions over a period of 10 days. The headspace of the model latrines were also sampled and analyzed to determine the gas phase concentration of perfume ingredients in the latrines.

Panelists were exposed to the odour of the three model latrines. The odour of two latrines were composed of perfumes Jasmin E and Floral V released from cellulosic pads in addition to the malodour Mukuru reconstitution delivered by forced evaporation systems as described in the previous example. The odour of the third model latrine was composed of the malodour alone in addition the blank cellulosic pad. The Cellulosic pads were 10.8cm X 7.3 cm X 0. 15cm, and were loaded with 2.2g of a mixture of 60% perfume oil and 40% isopropyl myristate (IPM). The pads were placed on a scale that equipped each latrine. The scales were connected to a computer to monitor every 5min the loss of mass of each pad. Time of implementation of the pads was time 0. Sensory analysis and headspace analysis were conducted according to the methods described in the previous example.

Referring to Figure 18 (reporting the perceived intensity of fecal malodour and reported pleasantness score), both the Floral V and Jasmine E formulations decreased the fecal character of the latrine malodour and increased the pleasantness. The highest performance for both formulations was obtained at the start of the experiment (day 0), where the pleasantness was the highest and the fecal character the lowest.

The performance of the formulations tended to decrease as a function of time at 25°C, and clearly decreased at 40°C. For Floral V, the pleasantness dropped under the neutral limit (5) to reach the negative valence (I don't like) after 10 days at 25°C, and after 4 days at 40°C. In fact, an inversion between the pleasantness and the fecal character was observed. The Jasmine E formulation demonstrated a better performance in both climates as the pleasantness remained in the positive valence (I like) during the survey and the fecal character ratings were lower than those observed with the Floral V formulation. Unlike the Floral V formulation, no inversion was observed for the Jasmine E formulation. Fluctuations were not related to the malodour, as the associated ratings were remarkably stable over time.

Analysis of the headspace of the test latrines revealed the variations of the gas phase concentration for all the compounds of the Floral V formulation and for selected compounds for the Jasmin E formulation. Figure 19, shows the evolution (i.e. headspace concentration) of antagonist molecules shared by both test formulations.

Referring to Figure 19, dihydrolinalol concentrations rapidly decreased to reach the olfactory detection threshold (ODT) on day 4 at 40°C and on day 5 at 25°C. Dihydrolinalol is an antagonist of butyric acid receptors. These data suggest that the suppression of the butyric acid perception should be minor or null when the gas phase concentration of Dihydrolinalol is below its ODT.

Violet AT and Isoraldeine 70P demonstrated similar decreases in headspace concentration. Here, their gas phase concentrations were stable at 25°C whereas, at 40°C, they started higher and decreased faster.

LILYFLORE^{®} was stable over the period of experiments but in higher concentration at 40°C compared to 25°C. Here, these data suggest, an increment of temperature helped to release LILYFLORE^{®}. All the antagonist compounds except Dihydrolinalol were in percievable amount in the air over the period of experiments.

**Table 16. The concentration at which certain MOCs reduce the percieved intensity of indol, DMTS, p-cresol and butyric acid.**

| | Indole | | DMTS | | p-cresol | | Butyric acid | |
|---|---|---|---|---|---|---|---|---|
| | Reducti on | Dyn Conc (ug/l ) | Reducti on | Dyn Conc (ug/l) | Reducti on | Dyn Conc (ug/l) | Reducti on | Dyn Conc (ug/l) |
| LILYFLOR E^{®} | 81 | 2.06 E-01 | 64 | 1.26E-01 | 76 | 2.20E-01 | 58 | 3.51E-02 |
| Violet AT | 72 | 1.33 E-01 | 81 | 1.51E-01 | 85 | 1.33E-01 | 55 | 1.23E-01 |
| Isoraldeine 70P | 79 | 5.42 E-01 | 69 | 2.16E-01 | 49 | 6.74E-02 | 25 | 6.74E-02 |
| Dihydrolina lol | NA | NA | NA | NA | NA | NA | 64 | 7.60E-01 |
| Aldehyde C11 undecylenic | 57 | 6.68 E-02 | 62 | 6.67E-02 | NA | NA | NA | NA |
| Citronellol BJ | 65 | 2.52 E-01 | NA | NA | 61 | 1.10E-01 | 55 | 9.47E-02 |
| Alpha damascone | NA | NA | NA | NA | 62 | 4.13E-02 | 43 | 4.10E-02 |
| Delphone | NA | NA | 67 | 4.60E-02 | 64 | 4.88E-02 | NA | NA |
| Hexylcinna mic aldehyde | NA | NA | NA | NA | 39 | 1.34E+ 00 | 26 | 1.34E+ 00 |
| Phenylethyl alcohol | 46 | 5.38 E-01 | 55 | 1.03E+ 00 | 49 | 3.18E-01 | NA | NA |
| Rosinol Cryst | NA | NA | 38 | 6.59E-01 | NA | NA | NA | NA |
| Zestover | 82 | 3.48 E-01 | 78 | 3.80E-01 | 66 | 6.07E-02 | 68 | 8.09E-02 |
| Benzyl acetate | NA | NA | NA | NA | NA | NA | 54 | 1.31E+ 00 |

Using the performance Floral V formulation at 40°C as an example, referring to Table 17 below, at Day 0, when the performance of the formulation was greatest (for both fecal malodor reduction and an increased perception of pleasantness), the headspace concentration of most of the ingredients were at or above the effective antagonist concentration for at least one malodour target. At day 2, the headspace concentration of the ingredients declined, with only one ingredient being at or above the effective antagonist concentration for at least one malodour target. Four ingredients were close to the effective antagonist concentration for at least one malodour target, and seven ingredients were below the effective antagonist concentration for at least one malodour target. A corresponding decline in performance of the formulation was observed (Figure 18). An inversion between the pleasantness and the fecal character was observed (Figure 18), and the headspace concentration of all the ingredients were below the effective MOC concentration for at least one malodour target.

**Table 17. Headspace concentration of MOCs.**

| | Headspace Concentration (µg/l) | | |
|---|---|---|---|
| | Floral V 40°C Day 0 | Floral V 40°C Day 2 | Floral V 40°C Day 6 |
| LILYFLORE^{®} | 1.00E-02 | 1.00E-02 | 1.00E-02 |
| Violet AT | 1.00E-01 | 6.00E-02 | 1.00E-02 |
| Isoraldeine 70P | 1.00E-01 | 7.00E-02 | 2.00E-02 |
| Dihydrolinalol | 8.00E-01 | 5.00E-03 | 3.00E-04 |
| Aldehyde C11 undecylenic | 6.00E-02 | 2.00E-02 | 0.00E+00 |
| Citronellol BJ | 7.00E-01 | 3.00E-01 | 1.00E-02 |
| Alpha damascone | 4.00E-02 | 2.00E-02 | 1.00E-03 |
| Delphone | 1.00E-01 | 4.00E-03 | 0.00E+00 |
| Hexylcinnamic aldehyde | 2.00E-03 | 3.00E-03 | 3.00E-03 |
| Phenylethyl alcohol | 1.00E+00 | 9.00E-02 | 1.00E-02 |
| Rosinol Cryst | 2.00E-02 | 2.00E-02 | 1.00E-02 |
| Zestover | 6.00E-02 | 2.00E-04 | 8.00E-05 |

In another study using latrines in Durban (South Africa), and Pune (India), the performances of the following two formulations were tested: Floral V Jasmin E and Citrus 259389 B (as described in Table 8). The formulations were incorporated into cellulose pads. The panel of participants were trained on-site to evaluate the performance of the test compositions over a period of 3 days. The headspace of the latrines were also sampled and analyzed to determine the gas phase concentration of perfume ingredients in the latrines.

Odour evaluation was performed by 11 subjects. The test formulations were diluted in isopropyl myristate (IPM; 60% oil, 40% IPM) and loaded on plain cellulose pads (10.8cm X 7.3cm X 0.15cm) at 42% w/w dry substrate. One to two pads were used per latrine according to the resulting intensity of the perfume when implemented. We used three latrines (three replicates) per formulation in both countries. In Durban, Jasmin E and Floral V were implemented in six private and individual ventilated pit-latrines and Citrus 259389 B (as described in Table 7) was implemented in three ventilated improved pit-latrines of a community ablution block. In Pune, each test formulation was implemented in three toilets of a dedicated ablution block.

The subjects evaluated the olfactory stimuli with our web-questionnaire developed in house and available on the internet at the following link: http://www.pacchiani.ch/firmenick/. They were asked to rate on a linear scale (from 0 to 100), the pleasantness from "I don't like" to "I like", the intensity from "no odour" to "very strong", the fecal character from "not fecal" to "very fecal". They were able to add comments at the end of the questionnaire.

The odour of the latrines was evaluated before and after the implementation of the pads. A first evaluation was performed in the afternoon to establish the baseline. The pads were then implemented and a second evaluation was performed 10-30min after the implementation. A third and a fourth evaluation was performed in the morning the next days after the implementation. For the evaluation, the participants were asked to enter the toilet one by one. The toilets were used as usual.

*Headspace Analysis:* The day following the implementation of the test formulations, the air of two toilets treated with the test formulations was sampled. The air was pumped at 1L/min through OASIS HLB 1g cartridges that were suspended to the walls of the latrines. One cartridge was placed near the ground at 0.15-0.3m height and a second one was placed at 1.5-1.7m height. The total volume pumped was 87L to 100L. The analysis and the quantifications were achieved according the standard protocols. To determine whether the test formulations changed significantly the pleasantness and the fecal character ratings, the Wilcox signed-rank test was used on the data of each toilet comparing the ratings before and after the implementation of the test formulations. The data obtained from the evaluations performed after the implementation, were pooled. Data of evaluations coming from toilets where the pad was stolen, displaced or removed were discarded.

Referring to Figures 20 and 21, the use of Floral V, Jasmine E, or Citrus 259389 B, via incorporation into cellulose pads significantly increased the pleasantness ratings in most latrines used tested (Figure 20). Moreover, in most cases, both formulations increased the pleasantness to a positive valence even in latrines with unpleasant baseline odours (Figure 20). The increase of pleasantness was related to a significant decrease of the fecal character (Figure 21), showing the malodour suppression effect of formulations containing antagonists of fecal malodourant molecules.

The fecal malodour observed was not constant across all the latrines tested. For example, in Pune, the level of the fecal malodour was very low and more similar among the pool of latrines compared to the fecal malodour observed in the latrines tested in Durban (Figure 21).

In Pune, the test formulations were implemented in three public ablution blocks composed of about 10 flush toilets. They were well maintained and cleaned several times a day. The ventilation was insured by opened windows in each toilet. In contrast, the latrines in Durban were dirty individual pit-latrines that were poorly maintained. For some latrines the pit was full and the ventilation port was missing. This can explain the variability of the malodour level in the different toilets. Examples are latrine N°2 treated with Jasmin E and in latrine N°1 treated with Floral V, the formulations hardly increased the average pleasantness that was not stable in time (Figure 22). Moreover, fecal malodour was not the only contributor to the unpleasant odour of both latrine: the malodour was also urine and chicken excrement. When the malodour was strong in latrines equipped with proper ventilation system, the test formulations decreased the malodour and this effect was stable in time (Figure 23). Figure 24 reports the results of clean and well maintained toilets in Africa and in India. Intriguingly, when the malodour was very strong in term of fecal character and intensity, the test formulations decreased the fecal character but also the total intensity (Figure 23), showing that the effect of malodour suppression is not associated with perfume overpowering the malodour.

Analysis of the headspace of the latrines revealed that the MOC molecules α-ionone, isoraldeine, LILYFLORE^{®}, dihydrolinalol were found in significant amounts in the air sampled in Pune and in Durban. The gas phase concentrations detected exceeded their respective olfactory detection thresholds (ODT) determined in separately, namely: 5.08×10⁻⁴, 1.92×10⁻⁴, 1.28×10⁻⁴, 1.37×10⁻³ µg/L, respectively (Figure 25). Surprisingly, similar concentrations were observed at low and high height in every toilet except in toilet N°3 in Durban (Figure 25).

The headspace analysis shows a certain degree of homogeneity despite the lack of airflow control. The exception of toilet N°3 may be explained by the fact that the cartridge at high height was too close from the pad during the sampling. Moreover, the gas phase concentrations were similar across both countries. However, two pads per toilets were used in Pune, suggesting that the ventilation rate was higher in Pune than in Durban.

The analysis revealed also that the profile of compounds concentrations obtained on the field were similar to that found in the model latrines at a similar temperature (Figure 26). The temperatures were 28°C, 25°C and 25°C in Durban, Pune and in the model latrines respectively. The gas phase concentrations were about two times higher in the model latrines than the concentrations observed in the latrines in Durban and India, suggesting that the ventilation rates in actual latrines were higher than in the model latrines.

Taken together, these data demonstrate a correlation between model latrines and field latrines, validating a step further the use of model latrines to make experiments in controlled conditions. Moreover, The Jasmine E formulation appeared to perform longer than the Floral V formulation in suppressing the toilet malodour reconstitution at 25°C and 40°C. Headspace analysis revealed that the gas phase concentrations of MOC compounds were similar comparing both perfumes and that top note compounds are not involved in the suppression of the malodour, challenging their presence in those formulations.

Formulations containing antagonists of fecal malodour (Jasmine E, Floral V, Citrus 259389 B) increased the pleasantness of toilets odour by decreasing the fecal character in different and challenging environments. However, the limit of performance was reached in dirty toilets with no ventilation and pit full, environments that are not targeted in this study. Furthermore, the MOC were in a significant amount even in challenging environment and that the suppression effect of the fecal character was not due to perfume overpowering the malodour.

### Example 15

### Synergistic effect of certain malodour receptor antagonists according to some aspects of the present invention

The performance of a test formulation containing LILYFLORE^{®}, Violet AT and Isoraldeine to counteract the fecal malodour perceived from a fecal malodour reconstitution was tested. The amount of each single ingredient in the test formulation was the same as the corresponding concentration of the ingredient that was incorporated into the Floral compositions. Separate control formulations were also included, comprising LILYFLORE^{®}, Violet AT and Isoraldeine separately, at the same concentration as each single ingredient in the test formulation. The sensory evaluation was blind, and performed using olfactometers and a set of more than 30 participants evaluating blind. In parallel, the headspace concentration of the ingredients from the test and three control formulations was determined. The results are shown in Table 18 and Figure 27 below.

**Table 18. Gas phase concentrations and attribute scores for the test and control formulations evaluated in combination with a fecal reconstitution.**

| | Fecal | | | | Freshness | | Pleasantnes s | | Conc µg/l air | ANOV A |
|---|---|---|---|---|---|---|---|---|---|---|
| | Scor e | CI | Scor e left | % Red n | Scor e | CI | Score | CI | | |
| Fecal Reconstitution | 8.6 | 0. 3 | 100 | 0 | 1.1 | 0. 3 | 1.0 | 0.2 | 0.021/0.02 1 | A |
| Control 1: LILYFLORE ^{®} | 3.8 | 0. 6 | 44 | 56 | 4.4 | 0. 6 | 4.8 | 0.5 | 1.36/1.22 | B |
| Control 2: Violette AT | 3.6 | 0. 6 | 42 | 58 | 4.7 | 0. 6 | 5.0 | 0.6 | 1.09/0.98 | B |
| Control 3: Isoraldeine | 3.6 | 0. 6 | 42 | 58 | 4.8 | 0. 6 | 5.1 | 0.5 | 0.96/0.86 | B |
| Test Formulation | 2.6 | 0. 6 | 30 | 70 | 5.4 | 0. 6 | 5.7 | 0.6 | 3.41/3.47 | C |

Taken together, these data demonstrate that the test formulation comprising a mixture of LILYFLORE^{®}, Violet AT and Isoraldeine was better at reducing the fecal score, and therefore the perception of fecal malodor, and also increasing the pleasantness and freshness score, compared to control formulations containing the single ingredients tested at their same concentration within the mixture.

The performance of another test formulation containing LILYFLORE^{®}, Violet AT and dihydrolinalol to counteract the fecal malodour perceived from a fecal malodour reconstitution was tested. Separate control formulations were also included, comprising LILYFLORE^{®}, Violet AT and dihydrolinalol separately, at the same concentration as each single ingredient in the test formulation. The sensory evaluation was blind, and performed using olfactometers and a set of more than 30 participants evaluating blind. In parallel, the headspace concentration of the ingredients from the test and three control formulations was determined. The results are shown in Table 19 and Figure 28 below.

**Table 19, Gas phase concentrations and attribute scores for the test and control formulations evaluated in combination with a fecal reconstitution.**

| | Fecal | | | | Freshness | | Pleasantness | | Conc µg/l air | ANOVA |
|---|---|---|---|---|---|---|---|---|---|---|
| | Score | CI | Score left | % Redn | Score | CI | Score | CI | | |
| Fecal reconstitution | 8.6 | 0.2 | 100 | 0 | 1.1 | 0.3 | 1.0 | 0.3 | 2.1×10⁻² | A |
| Control 1: dihydrolinalol | 5.6 | 0.7 | 65 | 35 | 3.9 | 0.6 | 3.5 | 0.7 | 1.36 | B |
| Control 2: | 4.7 | 0.7 | 55 | 45 | 4.0 | 0.6 | 4.1 | 0.8 | 1.13 | C |
| LILYFLORE^{®} | | | | | | | | | | |
| Control 3: Violet AT | 4.1 | 0.7 | 47 | 53 | 4.5 | 0.6 | 4.8 | 0.7 | 0.96 | C |
| Test Formulation | 3.1 | 0.6 | 35 | 65 | 5.3 | 0.6 | 5.5 | 0.7 | 3.40 | D |

Taken together, these data demonstrate that the test formulation comprising a mixture of LILYFLORE^{®}, Violet AT and dihydrolinalol was better at reducing the fecal score, and therefore the perception of fecal malodor, and also increasing the pleasantness and freshness score, compared to control formulations containing the single ingredients tested at their same concentration within the mixture.

The performance of another test formulation containing LILYFLORE^{®}, isoraldeine, Violet AT and dihydrolinalol to counteract the fecal malodour perceived from a fecal malodour reconstitution was tested. Separate control formulations were also included, comprising LILYFLORE^{®}, Violet AT, isoraldeine and dihydrolinalol separately, at the same concentration as each single ingredient in the test formulation. The sensory evaluation was blind, and performed using olfactometers and a set of more than 30 participants evaluating blind. In parallel, the headspace concentration of the ingredients from the test and three control formulations was determined. The results are shown in Table 20 and Figure 29 below.

**Table 20. Gas phase concentrations and attribute scores for the test and control formulations evaluated in combination with a fecal reconstitution.**

| | Fecal | | | | Freshness | | Pleasantness | | Conc gg/l air | ANOVA |
|---|---|---|---|---|---|---|---|---|---|---|
| | Score | CI | Score left | % Redn | Score | CI | Score | CI | | |
| Fecal reconstitution | 8.6 | 0.2 | 100 | 0 | 1.1 | 0.3 | 1.0 | 0.3 | 2.1×10⁻² | A |
| Control 1: dihydrolinalol | 6.1 | 0.7 | 70 | 30 | 3.2 | 0.7 | 3.2 | 0.6 | 1.12 | B |
| Control 2: Violete AT | 4.9 | 0.8 | 56 | 44 | 4.1 | 0.8 | 4.2 | 0.7 | 7.47 | C |
| Control 3: LILYFLORE^{®} | 4.8 | 0.8 | 55 | 45 | 3.9 | 08 | 3.8 | 0.6 | 9.34 | C |
| Control 4 : Isoraldeine | 4.4 | 0.8 | 51 | 49 | 4.6 | 0.6 | 4.5 | 0.8 | 6.53 | C |
| Test Formulation | 3.1 | 0.7 | 36 | 64 | 5.1 | 0.7 | 5.5 | 0.7 | 3.45 | D |

Taken together, these data demonstrate that the test formulation comprising a mixture of LILYFLORE^{®}, Violet AT, isoarldeine and dihydrolinalol was better at reducing the fecal score, and therefore the perception of fecal malodor, and also increasing the pleasantness and freshness score, compared to control formulations containing the single ingredients tested at their same concentration within the mixture.

## Claims

1. A malodour counteracting composition comprising
a) from 2 to 85 wt% of an active amount of a malodour receptor antagonist system comprising at least one ingredient selected from Table 1;
b) from 15 to 98 wt% of a functional perfume accord comprising at least two ingredients selected from Table 2, derivatives and mixtures thereof;
**characterized in that** the malodour receptor antagonist system comprises (2,5-dimethyl-2,3-dihydro-1H-inden-2-yl)methanol, and the functional perfume accord comprises citral, isoraldeine and α-ionone.

2. The composition according to claim 1, **characterized in that** (2,5-dimethyl-2,3-dihydro-1H-inden-2-yl)methanol is present at least at 2%, alternatively, at 3% of the composition.

3. The malodour counteracting composition according to any one of claims 1 to 2, **characterized in that** it further comprises an encapsulating materials or other materials to form an emulsion, a dispersion, a micro-emulsion, a miniemulsion, a gel, a micro-gel, a microcapsule or a microparticle.

4. The malodour counteracting composition according to any one of claims 1 to 3, wherein the malodour receptor antagonist system comprises at least 3 ingredients selected from Table 1.

5. The malodour counteracting composition according to any one of claims 1 to 4, wherein the malodour counteracting composition further comprises a non-functional perfume accord comprising at least two perfuming ingredients.

6. A perfumed consumer product comprising an effective amount of a malodour counteracting composition as defined in defined in any one of claims 1 to 5.

7. The perfumed consumer product according to claim 6, selected from air care products, home care products and laundry care products.

8. The perfumed consumer product according to claim 6 or 7, **characterized in that** it is in the form of an aerosol and/or water-based air freshener spray, wick/reed air freshener, liquid electrical (plug-in) air freshener, a solid support air freshener, gel-based air freshener, membrane-containing air freshener, bleaching, cleaning, washing detergent powder, liquid all-pupose cleaner, specialty cleaner and liquid detergent.

## Patentansprüche

1. Unangenehmem Geruch entgegenwirkende Zusammensetzung, umfassend:
a) 2 bis 85 Gew.% einer aktiven Menge eines Unangenehmer-Geruch-Rezeptor-Antagonistsystems, umfassend wenigstens einen Inhaltsstoff ausgewählt aus Tabelle 1;
b) 15 bis 98 Gew.% an einem funktionellen Parfümakkord, umfassend wenigstens zwei Inhaltsstoffe ausgewählt aus Tabelle 2, Derivate und Mischungen davon;
**dadurch gekennzeichnet, dass** das Unangenehmer-Geruch-Rezeptor-Antagonistsystem (2,5-Dimethyl-2,3-dihydro-1H-inden-2-yl)methanol umfasst und der funktionelle Parfümakkord Citral, Isoraldein und α-Ionon umfasst.

2. Zusammensetzung nach Anspruch 1, **dadurch gekennzeichnet, dass** (2,5-Dimethyl-2,3-dihydro-1H-inden-2-yl)methanol in wenigstens 2 %, alternativ 3 % der Zusammensetzung vorhanden ist.

3. Unangenehmem Geruch entgegenwirkende Zusammensetzung nach einem der Ansprüche 1 bis 2, **dadurch gekennzeichnet, dass** sie des Weiteren verkapselnde Materialien oder andere Materialien zur Bildung einer Emulsion, einer Dispersion, einer Mikroemulsion, einer Miniemulsion, eines Gels, eines Mikrogels, einer Mikrokapsel oder eines Mikropartikels umfasst.

4. Unangenehmem Geruch entgegenwirkende Zusammensetzung nach einem der Ansprüche 1 bis 3, wobei das Unangenehmer-Geruch-Rezeptor-Antagonistsystem wenigstens 3 Inhaltsstoffe ausgewählt aus Tabelle 1 umfasst.

5. Unangenehmem Geruch entgegenwirkende Zusammensetzung nach einem der Ansprüche 1 bis 4, wobei die unangenehmem Geruch entgegenwirkende Zusammensetzung des Weiteren einen nicht-funktionellen Parfümakkord umfasst, der wenigstens zwei Parfürmierinhaltsstoffe umfasst.

6. Parfümiertes Konsumprodukt, umfassend eine effektive Menge einer unangenehmem Geruch entgegenwirkenden Zusammensetzung wie in einem der Ansprüche 1 bis 5 definiert.

7. Parfümiertes Konsumprodukt nach Anspruch 6 ausgewählt aus Luftbehandlungsprodukten, Hauspflegeprodukten und Wäschepflegeprodukten.

8. Parfümiertes Konsumprodukt nach Anspruch 6 oder 7, **dadurch gekennzeichnet, dass** es in Form von Aerosol und/oder Lufterfrischungsspray auf Wasserbasis, Lufterfrischer mit Docht/Rohrblatt, flüssigem elektrischen (Einsteck)-Lufterfrischer, Lufterfrischer mit festem Träger, Lufterfrischer auf Gelbasis, membranhaltigem Lufterfrischer, Bleichmittel, Reinigungsmittel, Waschmittelpulver, flüssigem Allzweckreiniger, Spezialreiniger und Flüssigwaschmittel vorliegt.

## Revendications

1. Composition contre les mauvaises odeurs comprenant
a) de 2 à 85 % en poids d'une quantité active d'un système d'antagonistes des récepteurs de mauvaises odeurs comprenant au moins un ingrédient choisi dans le tableau 1 ;
b) de 15 à 98 % en poids d'un accord de parfum fonctionnel comprenant au moins deux ingrédients choisis dans le tableau 2, des dérivés et des mélanges correspondants ;
**caractérisée en ce que** le système d'antagoniste des récepteurs de mauvaises odeurs comprend du (2,5-diméthyl-2,3-di-hydro-1H-indén-2-yl)méthanol, et l'accord de parfum fonctionnel comprenant du citral, de l'isoraldéine et de la α-ionone.

2. Composition selon la revendication 1, **caractérisée en ce que** le (2,5-diméthyl-2,3-dihydro-1H-indén-2-yl)méthanol est présent à raison d'au moins 2 %, en variante, à raison de 3 % de la composition.

3. Composition contre les mauvaises odeurs selon l'une quelconque des revendications 1 à 2, **caractérisée en ce qu'**elle comprend en outre un matériau d'encapsulation ou d'autres matériaux pour former une émulsion, une dispersion, une microémulsion, une miniémulsion, un gel, un microgel, une microcapsule ou une microparticule.

4. Composition contre les mauvaises odeurs selon l'une quelconque des revendications 1 à 3, le système d'antagoniste des récepteurs de mauvaises odeurs comprenant au moins 3 ingrédients choisis dans le tableau 1.

5. Composition contre les mauvaises odeurs selon l'une quelconque des revendications 1 à 4, la composition contre les mauvaises odeurs comprenant en outre un accord de parfum non fonctionnel comprenant au moins deux ingrédients parfumants.

6. Produit de consommation parfumé comprenant une quantité efficace d'une composition contre les mauvaises odeurs telle que définie dans l'une quelconque des revendications 1 à 5.

7. Produit de consommation parfumé selon la revendication 6, choisi parmi des produits d'assainissement de l'air, des produits d'entretien ménager et des produits d'entretien de blanchisserie.

8. Produit de consommation parfumé selon la revendication 6 ou 7, **caractérisé en ce qu'**il est sous la forme d'un aérosol et/ou d'un spray désodorisant à base d'eau, d'un désodorisant à mèche/à tiges, d'un désodorisant électrique (enfichable), d'un désodorisant à support solide, d'un désodorisant à base de gel, d'un désodorisant contenant une membrane, d'une poudre de détergent pour blanchiment, nettoyage, lavage, d'un agent de nettoyage tous usages liquide, d'un agent de nettoyage spécialisé et d'un détergent liquide.
